# EUROPEAN PATENT SPECIFICATION

(11) **EP 3 432 939 B1**
(45) Date of publication and mention of the grant of the patent: **01.05.2024**
(21) Application number: 17715260.0
(22) Date of filing: 23.03.2017
(51) Int. Cl.: A61L 27/18, C08L 67/04, C08L 71/02, A61L 27/10, A61L 27/12, A61L 27/26, A61L 27/54, A61L 27/56

(54) **SCAFFOLDING MATERIAL, METHODS AND USES**
GERÜSTMATERIAL, VERFAHREN UND VERWENDUNGEN
MATÉRIAU D'ÉCHAFAUDAGE, PROCÉDÉS ET UTILISATIONS

(30) Priority: 24.03.2016 GB 201605122; 13.04.2016 GB 201606395
(43) Date of publication of application: 30.01.2019
(73) Proprietor: Heraeus Medical GmbH, 61273 Wehrheim (DE)
(72) Inventor: LEONARDI, Antonio, Nottingham Northamptonshire NG90 6BH (GB); COX, Helen C., Nottingham Nottinghamshire NG90 6BH (GB); CAREY, Siobhan, Nottingham Northamptonshire NG90 6BH (GB); MATTHEWS, Charles, Nottingham Northamptonshire NG90 6BH (GB); LOMAS, Alexander, Nottingham Northamptonshire NG90 6BH (GB); SHAKESHEFF, Kevin M, Nottingham Northamptonshire NG90 6BH (GB); QUIRK, Robin A, Nottingham Northamptonshire NG90 6BH (GB)
(74) Representative: Barker Brettell LLP
(86) International application number: PCT/GB2017/050815
(87) International publication number: WO 2017/163072

(56) References cited:
- EP-A2- 2 125 048
- WO-A1-2015/019109
- WO-A2-2010/100506

## Description

The invention relates to scaffolds, and to the use of such scaffolds in tissue and bone repair, and delivery systems to deliver an agent to a target site in a subject.

### Background

Within the field of regenerative medicine there are many opportunities for new clinical procedures that stimulate and support tissue repair. Examples of clinical opportunities include regeneration of cardiac muscle after an infarction, induction of bone growth in spinal fusion, healing of diabetic foot ulcers and limitation or, perhaps, reversal of damage due to stroke. Examples of tissues where treatment could facilitate healing are brain tissue, liver tissue and pancreatic tissue, amongst others.

One area where tissue healing is important is bone healing, for example for people with bone disorders. Bone healing is a physiological process in which the body facilitates the repair of the bone after an external injury, infection, surgical intervention or a disease. The physiological healing process can require very long periods and in many cases, it cannot re-establish the original bone properties. For this reason, therapies that accelerate and improve bone healing are of vital importance for people with bone disorders. Usually, these therapies present osteoconductive, osteoinductive, and osteogenic approaches.

In the majority of osteoconductive approaches, a variety of substitutes like gold, stainless steel, titanium, natural/synthetic polymers and ceramics have been tried. The main concerns with the use of these materials for bone reconstruction were their poor ability to vascularise, integrate, and undergo remodelling. This may result in structural failure of the implant under load or pathological changes in the surrounding bone, as seen in stress shielding. The other issues are inflammatory scarring, neoproliferative reaction in the adjacent tissues and infection. Because of their high osteoinductive potential and remodelling characteristics, bioactive substitutes have been used with promising results. This led to the evolution of tissue engineering techniques (biologically enhanced allografts, cell-based therapies, and gene-based therapies) to treat bone disorders. Tissue engineering has been defined as the application of scientific principles to the design, construction, modification, and growth of living tissue using biomaterials, cells, and factors alone and in combination. It involves the use of osteoconductive biomaterial scaffolds, with osteogenic cell populations and osteoinductive bioactive factors. All these approaches have the potential to significantly increase our ability to treat diseases for which no effective treatment currently exists.

Scaffolds can provide an appropriate mechanical environment, architecture and surface chemistry for angiogenesis and tissue formation. The localisation of regenerative agents, such as growth factors, can also be achieved using scaffolds. The use of scaffolds as drug or cell delivery systems has great potential but is also very challenging due to the need to tailor the porosity, strength and degradation kinetics of the scaffolds to the tissue type whilst achieving the appropriate kinetics of release of agents, such as proteins that act as growth factors or cells. A further complication in the use of scaffolds as delivery systems for in vivo repair and/or regeneration is the issue of the route of administration. In many clinical examples the site of tissue requiring repair is either difficult to access (e.g. within the brain for stroke therapies or cardiac muscle for post infarction treatment) or of unknown size and shape. Therefore, there is a need for improved injectable scaffolds that can be administered via minimally invasive procedures.

In broad terms, a scaffold is typically either a pre-formed water-insoluble matrix, with large interconnected pores or a hydrogel. Such scaffolds are implanted into a patient for augmented in vivo tissue repair and/or regeneration.

In terms of implantation, the pre-formed water-insoluble matrices must be shaped to fill a cavity within the body, requiring knowledge of the cavity dimensions and limiting the shape of cavity that can be filled. In addition, an invasive operation is required to deliver the scaffold.

In contrast, a number of hydrogel materials have been designed that can be delivered directly into the body through a syringe. The gel forms within the body following a trigger signal, for example a temperature change or UV light exposure. Such systems have the advantage that they can fill cavities of any shape without prior knowledge of the cavity dimensions. However, such hydrogels lack large interconnected porous networks and, hence, release of an agent from the gel is limited by poor diffusion properties. Furthermore, the poor mechanical strength of hydrogels means they are often unable to withstand the compressive forces applied in use, furthermore this can result in undesirable delivery properties, as agents in the gels can be in effect squeezed out of the hydrogel.

Resorbable putty or resorbable pastes that solidify after body application, are promising approaches. This area has been widely researched both academically and industrially, with several products such as C-Graft Putty^{™}, Grafton^{®} already having been commercialised. The major obstacles in the success of such approaches are the successful delivery and retention of materials to the required site of action, as well as their malleability before the surgery.

WO2008093094 and WO2004084968
describe compositions and methods for forming tissue scaffolds from polymer pellets, such as PLGA and PLGA/PEG polymer blends. Such scaffolds have been developed to be capable of moulding or injection prior to setting *in situ* at the site of tissue repair. The setting *in situ* can be achieved by, for example, exploiting and tuning the glass transition temperature of the pellets for interlinking/crosslinking of the pellets at body temperature. Interlinking events can also be facilitated by non-temperature related methods, such as by plasticisation by solvents. A porous structure is achieved by leaving gaps between the pellets and optionally further providing porous polymer pellets. The resulting scaffolds maintain a high compressive strength that is useful in tissue repair, especially for connective tissues such as bone, whilst also maintaining porosity useful for cell growth and agent delivery. However, an aim of the present invention is to provide improved compositions, methods and processes for forming scaffold material for use in tissue repair.

WO2010100506 discloses a scaffold material obtained by mixing polymer microparticles, which can be made of a natural or a synthetic polymer, with a therapeutic agent and a liquid carrier. The mixture is suspended in the liquid carrier and subjected to a setting step wherein the discrete particles interact with each other to form a solid scaffold with the therapeutic agent encapsulated amongst the scaffold of polymer microparticles. The carrier might contain one or more plasticizers. The plasticizer may, for example, be PEG, polypropylene glycol, poly(lactic acid) or (poly(glycolic acid) or a copolymer thereof, polycaprolactone, and low molecule weight oligomers of these polymers or conventional plasticisers, such as, adipates, phosphates, phthalates, sebacates, azelates and citrates.

An aim of the present invention is to provide improved methods and processes for forming scaffold material for use in tissue repair.

### SUMMARY OF INVENTION

The scope of this invention is defined by the claims. Any "embodiment" or "example" which is disclosed in the description but is not covered by the claims should be considered as presented for illustrative purpose only. Embodiments in the description relating to methods of treatment are not covered by the claims.

According to a first aspect of the present invention, there is provided a method of forming a scaffold material in accordance with claim 1 According to a second aspect of the claimed invention, there is provided a scaffold material for forming a scaffold according to claim 3. According to a third aspect of the claimed invention, there is provided a kit for use to form a scaffold from scaffold material according to claim 5.

Also described is a method of forming a scaffold material which is capable of setting in less than 5 minutes, wherein the scaffold material is provided in accordance with the method of the invention and wherein the plasticiser is provided in the carrier in a range of between about 4% and about 6% (w/v) of plasticiser.

Also described is a method of forming a scaffold material having a scaffold setting time of between about 5 and about 15 minutes, wherein the scaffold material is provided in accordance with the method of the invention, and wherein the plasticiser is provided in the carrier in a range of between about 2.5% and about 3.5% (w/v) of plasticiser.

Also described is a method of forming a scaffold material suitable for forming a scaffold having a 1^{st} order agent release kinetic, wherein the scaffold material is provided in accordance with the method of the invention, and wherein the agent is provided as a powder prior to blending with polymer to form the polymer microparticles of the scaffold material.

Also described is a scaffold material produced by the claimed method-Also described is a scaffold produced by any method of the description.

The current description describes resorbable scaffold material able to set at different times and at different temperatures. Such pastes may provide a scaffold support for tissue formation if used alone, or osteoinductive and osteogenic effects if used with drugs or bioactive substitutes, such as cells, de-cellularised matrix (DCM) and growth factors. The control of paste setting under different temperatures can be useful for injectable pastes. For example, if the setting occurs at body temperature (37°C), said pastes can be handled with no rush at room temperature before injection. The control of paste setting under different times can be useful for making putties. In fact, a paste that sets after few minutes can form a putty that, depending on the needs, can be differently shaped and administered. The description herein further provides the ability to control drug release by changing the formulation variables of particles size, agent loading method, polymer type, plasticiser type and concentration and blend composition.

### FIGURES

The invention will be exemplified with the following accompanying figures, by way of example only.
**Fig.1** Experimental conditions for a cohesion test: sieve mesh/tray with immersed aluminium foils and pastes.
**Fig.2** PLGA 50:50 (50-100µm MPs) mass loss after 15 sintering at room temperature or 37°C
Fig.3 75.6% w/w PLGA50:50, 5.2% w/w PEG400, 20% w/w SIM (300-400µm HME pellets) mass loss after 15 sintering at room temperature or 37°C
**Fig.4** 46.5% w/w PLGA 95:5, 3.25% w/w PEG400, 50% w/w CS (300-400µm HME pellets) mass loss after 15 sintering at room temperature or 37°C
**Fig.5** 46.5% w/w PLGA 95:5, 3.25% w/w PEG400, 50% w/w β-TCP (300-400µm HME pellets) mass loss after 15 sintering at room temperature or 37°C
**Fig.6** 75.6% w/w PLGA50:50, 5.2% w/w PEG400, 20% w/w SIM (300-400µm HME pellets) mass loss after sintering at different time points.
**Fig. 7** 6x12mm scaffolds
**Fig. 8** Mechanical properties of 6x12 cylindrical PLGA 50:50 (50-200 µm) scaffolds after 15 minutes and 2 hours sintering at either 32°C or 37°C. N=3±1SD
**Fig. 9** Mechanical properties of PLGA 50:50 (50-200 µm) scaffolds sintered with 3% TEC after 24 hours sintering at 37°C in either wet (immersed in PBS) or damp (sealed in a humidified bag) conditions. N=3±1SD
**Fig. 10** Young's modulus of PLGA/CS (50-200 µm) scaffolds over time at 37°C, with 24 hours values for damp sinter conditions (37°C, ≥90% humidity) and wet conditions (fully immersed in 37°C Phosphate buffered saline, PBS)
**Fig. 11****.** Schematic of experimental set up for viscosity measurements.
**Fig. 12** Distance flowed by putty at 45° in 60s at room temperature using different carrier:polymer ratios and varying concentrations of TEC or 10% ethanol.
**Fig. 13** Distance flowed by paste containing 50% CaSO4 and blank PLGA paste material at t=0 minutes at 45° in 60 seconds

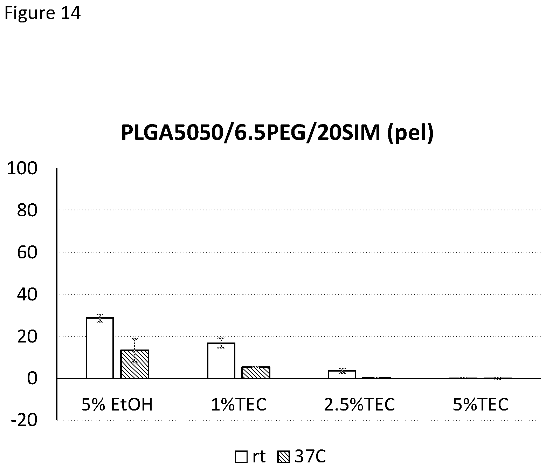

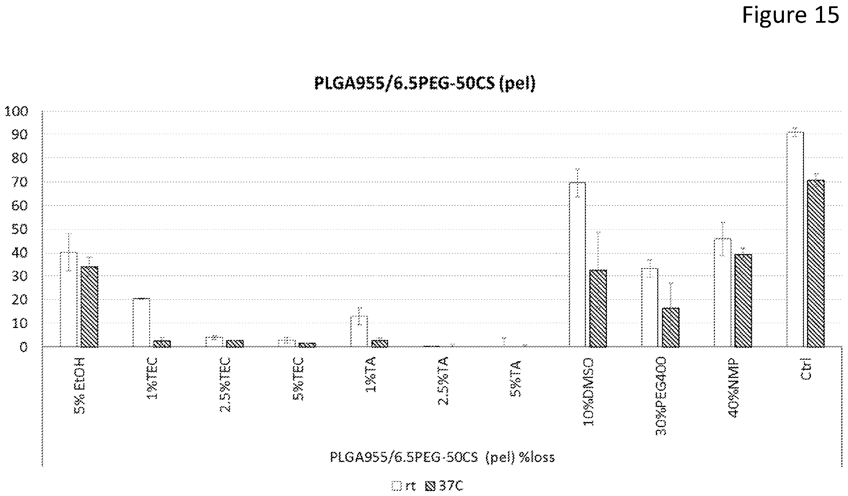

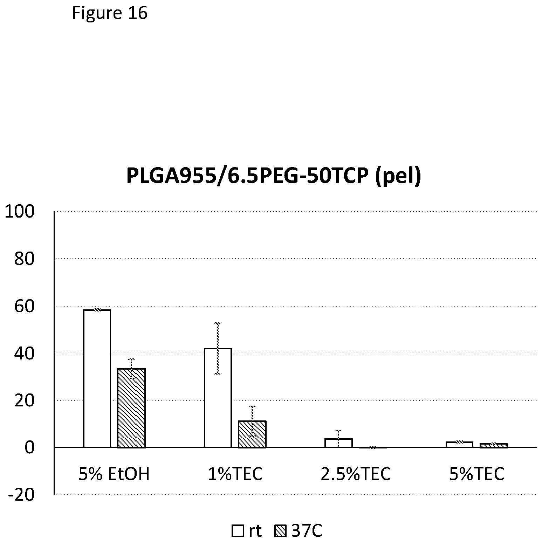

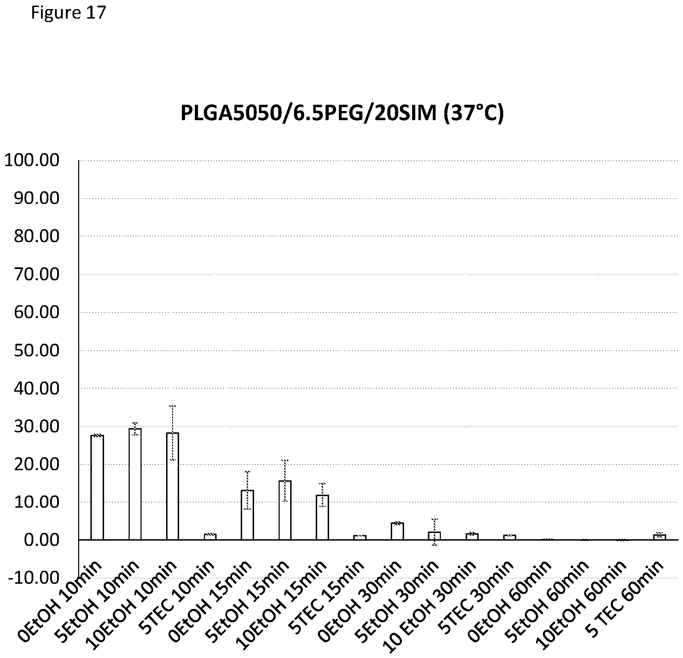

**Fig. 14** - 74.8% w/w PLGA50:50, 5.2% w/w PEG400, 20% w/w SIM (300-400µm HME pellets) mass loss after 15 sintering at room temperature or 37°C
**Fig. 15** - 46.75% w/w PLGA 95:5, 3.25% w/w PEG400, 50% w/w CS (300-400µm HME pellets) mass loss after 15 sintering at room temperature or 37°C
**Fig. 16** - 46.75% w/w PLGA 95:5, 3.25% w/w PEG400, 50% w/w β-TCP (300-400µm HME pellets) mass loss after 15 sintering at room temperature or 37°C
**Fig. 17** - 74.8% w/w PLGA50:50, 5.2% w/w PEG400, 20% w/w SIM (300-400µm HME pellets) mass loss after sintering at different time points.

### DETAILED DESCRIPTION

### DEFINITIONS

The term "scaffold material" is intended to refer to a composition that is capable of forming a scaffold, i.e. a pre-scaffold material. For example the scaffold material may comprise a composition that is capable of setting into a scaffold. The scaffold material itself may or may not have a structure of a scaffold until the scaffold material has formed the scaffold according to the methods herein. Reference to "a composition that is capable of forming a scaffold" may include the capability to form a scaffold with no further intervention/process steps or components. In an alternative embodiment, reference to "a composition that is capable of forming a scaffold" may include the capability to form a scaffold following further intervention/process steps according to the description herein and/or following addition of components according to the description herein.

The term "scaffold" (may be interchanged with the term "matrix") is understood to mean a solid mass of material having a 3-dimensional structure, which may for example be suitable to support cells. In embodiments of the description, the scaffold may be porous, having interconnected pores or gaps.

The term "room temperature" is intended to refer to a temperature of from about 15°C to about 25°C, such as from about 20°C to about 25°C.

The term "setting" herein is intended to refer to the act of solidifying, or otherwise fixing, the scaffold material into a solid scaffold. The setting may be actively promoted, for example by a change in conditions, such as temperature and/or pressure. In one embodiment, setting is achieved by sintering. In one embodiment, setting is achieved by addition of a setting agent and/or condition. In another embodiment, the setting of the scaffold material into a solid scaffold may be a passive step, for example the particles/pellets of the scaffold material may spontaneously interlink upon contact. This may be immediate interlinking upon contact, or for example over a period of time. **I**n one embodiment, the setting may be facilitated by leaching of plasticiser from the particles/pellets. Setting may be facilitated by administration/ implantation to a body or tissue.

The term "sintering" herein is intended to refer to a process of compacting and forming a solid mass of material by heat and/or pressure without melting it to the point of liquefaction. For example, sintering can happen naturally in mineral deposits.

The term "solidifying" or "solidify" herein is intended to refer to the change of state from a flowable state (for example, that may take the shape of a receptacle) to a non-flowable state where the pellets and/or particles of the scaffold material are interconnected and set in position relative to each other. For the purposes of the present description a putty or gel material may be considered a solidified material. The term "flowable" may include liquid or solid particles, pellets or powder that are not interconnected and are capable of flowing.

A "plasticiser" is a substance typically incorporated into a polymer to increase its flexibility, softness, distensibility or workability. Plasticizers can weaken the bonds holding the polymer molecules together and can have an effect on thermal and/or mechanical properties. The plasticiser may be a pharmaceutically acceptable plasticiser. The plasticiser may be ethanol.

The terms "inter-link" or "interlinking" are intended to refer to the particles/pellets becoming physically connected and held together (i.e. interacting and sticking together). Inter-linking may be achieved by covalent, non-covalent, electrostatic, ionic, adhesive, cohesive or entanglement interactions between the polymer pellets/particles or components of the polymer pellets/particles. The pellets/particles may be crosslinked/inter-linked.

### METHOD OF FORMING A SCAFFOLD

Described is a method of forming a scaffold material for controlled release of an agent *in situ,* the method comprising:
providing polymer microparticles;
providing an agent, wherein the agent is in a powder form;
mixing the polymer microparticles with the powder agent;
suspending the mixture in a liquid carrier to form a scaffold material that is a polymer microparticle suspension; and optionally
setting the scaffold material such that it sets into a solid scaffold of polymer microparticles, wherein the powder agent is encapsulated amongst the scaffold of polymer microparticles.

Described is a method of forming a scaffold for controlled release of an agent *in situ,* the method comprising:
providing polymer microparticles;
providing an agent, wherein the agent is in a powder form;
mixing the polymer microparticles with the agent;
suspending the mixture in a liquid carrier to form a scaffold material that is a polymer microparticle suspension; and
setting the scaffold material such that it sets into a solid scaffold of polymer microparticles, wherein the powder agent is encapsulated amongst the scaffold of polymer microparticles.

Advantageously, the provision of the agent in a powder form still allows scaffold formation, yet also allows a favourable release profile of the agent *in situ.* For example, the agent can become available as the powder form of the agent (such as crystals) is solubilised in the carrier and/or body fluid of the patient being treated. Therefore, a burst release of agent can be provided following implantation/injection of the scaffold, followed by a longer sustained release (i.e. a 1^{st} order kinetics release profile).

### The scaffold and scaffold material

The scaffold material may be for use in a method of treatment of the human or animal body by surgery or therapy or in a diagnostic method practised on the human or animal body. The scaffold material may be for pharmaceutical use or may be for use in cosmetic surgery.

In one embodiment, the scaffold of polymer microparticles is porous. The pores may be formed by voids within the polymer microparticles or by gaps between the polymer microparticles. In one embodiment, the pores are formed by voids within the polymer microparticles and by gaps between the polymer microparticles. The pores may be formed by the gaps which are left between polymer microparticles used to form the scaffold.

The scaffold may have a pore volume (i.e. porosity) of at least about 50%. The pores may have an average diameter of about 100 microns. The scaffold may have pores in the nanometre to millimetre range. The scaffold may have pores of about 20 to about 50 microns, alternatively between about 50 and 120 microns. In one embodiment, the scaffold has pores with an average size of 100 microns. The scaffold may have at least about 30%, about 40%, about 50% or more pore volume. In one embodiment, the porosity of the scaffold may be between 30% and 70%. In another embodiment, the porosity of the scaffold may be between 40% and 65%. In another embodiment, the porosity of the scaffold may be between 40% and 60%. In another embodiment, the porosity of the scaffold may be between 50% and 60%. The scaffold may have a pore volume of at least 90mm³ per 300mm³ of scaffold. In another embodiment, the scaffold may have a pore volume of at least 120mm³ per 300mm³ of scaffold. In another embodiment, the scaffold may have a pore volume of at least 150mm³ per 300mm³ of scaffold.

As the skilled man would appreciate, pore volume and pore size can be determined using microcomputer tomography (microCT) and/or scanning electron microscopy (SEM). For example, SEM can be carried out using a Philips 535M SEM instrument.

The polymer microparticle suspension may be injectable. The injectable scaffold material may be capable of setting (solidifying/self-assembling) upon/or after injection into a subject to form a scaffold. In one embodiment, the scaffold material is intended to be administered by injection into the body of a human or non-human animal. If the scaffold material is injected then the need for invasive surgery to position the scaffold is removed. The scaffold material may be sufficiently viscous to allow administration of the composition to a human or non-human animal, preferably by injection.

By using a scaffold material which solidifies/sets to form a scaffold after administration, a scaffold can be formed which conforms to the shape of where it is placed, for example, the shape of a tissue cavity into which it is placed. This overcomes a problem with scaffolds fabricated prior to administration which must be fabricated to a specific shape ahead of administration, and cannot be inserted through a bottle-neck in a cavity and cannot expand to fill a cavity.

The scaffold material may be arranged to be administered at room temperature. Therefore, the scaffold material may be viscous at room temperature. Alternatively, the scaffold material may be heated to above room temperature, for example to body temperature (about 37°C) or above, for administration. The scaffold material may be flowable or viscous at this temperature in order to aid its administration to a human or non-human animal.

The scaffold material may have a viscosity which allows it to be administered, using normal pressure (e.g. the pressure can be reasonably applied by the hand of an average person), from a syringe which has an orifice of about 4mm or less. The size of the orifice will depend on the medical application, for example, for many bone applications a syringe with an orifice of between about 2mm and about 4mm will be used, however, for other applications smaller orifices may be preferred. The term "normal pressure" may be pressure that is applied by a human administering the composition to a patient using one hand.

The scaffold material may be of sufficient viscosity such that when it is administered it does not immediately dissipate, as water would, but instead takes the form of the site where it is administered. Some or all of the carrier and agent may dissipate from the scaffold over time. In one embodiment, the scaffold material is sufficiently viscous that when administered the injectable scaffold material remain substantially where it is injected, and does not immediately dissipate. In one embodiment, the scaffold forms, or is arranged to form, before there has been any substantial dissipation of the scaffold material. More than about 50%, 60% 70%, 80% or 90% by weight of the scaffold material provided, such as injected, into a particular site may remain at the site and form a scaffold at that site.

The polymer microparticles may be capable of interlinking and setting into a solid scaffold by sintering. The scaffold material may be capable of spontaneously solidifying when injected into the body due to an increase in temperature post administration (e.g. increase in the temperature from room temperature to body temperature). This increase in temperature may cause the scaffold material to interact to form a scaffold.

When the scaffold material solidifies to form a scaffold it may change from a suspension or a deformable viscous state to a solid state in which the scaffold formed is self-supporting and retains its shape. The solid scaffold formed may be brittle or more flexible depending on its intended application. The scaffold may be compressible without fracturing (for example a sponge consistency).

Solidification of the scaffold material (i.e. formation/setting of scaffold from the scaffold material) may be triggered by any appropriate means, for example, solidification may be triggered by a change in temperature, a change in pH, a change in mechanical force (compression), or the introduction of an interlinking, cross-linking, setting or gelling agent or catalyst.

In one embodiment, the solidification is triggered by plasticiser interaction with the polymer microparticles, such that they crosslink/inter-link to form the scaffold. In particular, the plasticiser may alter the surface chemistry of the polymer microparticles such that the surface Tg is decreased, thereby allowing the polymer microparticles to stick/crosslink/inter-link together.

In other words, the polymer microparticles may be particles, such as discrete particles, that can be set/solidified into a scaffold by a change in temperature, a change in pH, a change in mechanical force (compression), or the introduction of an interlinking, cross-linking agent, setting agent or gelling agent or catalyst.

The scaffold material may be cross linked by a variety of methods including, for example, physical entanglement of polymer chains, UV cross linking of acrylate polymers, Michael addition reaction of thiolate or acrylate polymers, thiolate polymers cross linked via vinyl sulphones, cross linking via succinimates of vinyl sulphones, cross linking via hydrazines, thermally induced gelation, enzymatic crosslinking (for example, the addition of thrombin to fibrinogen), cross linking via the addition of salts or ions (especially Ca²⁺ ions), cross linking via isocyanates (for example, hexamethylene diisocyanate).

The scaffold material comprises discrete particles, which are capable of interacting to form a scaffold. The interaction may cause the particles to cross link, wherein the particles become physically connected and are held together. Cross linking may be achieved by covalent, non-covalent, electrostatic, ionic, adhesive, cohesive or entanglement interactions between the particles or components of the particles.

In one embodiment, the discrete particles are capable of cross linking, such that the particles become physically connected and are held together. The particles may suitably be polymer microparticles that are capable of cross linking, such that the particles become physically connected and are held together.

A characteristic for the particles, to ensure a scaffold can be formed, may be the glass transition temperature (Tg). By selecting polymer microparticles that have a Tg above room temperature, at room temperature, (e.g. about 24°C), the polymer microparticles are below their Tg and behave as discrete particles, but when exposed to a higher temperature (e.g. in the body) the polymer microparticles soften and interact/stick to their neighbours. In one embodiment, polymer microparticles are used that have a Tg from about 25°C to 50°C, such as from about 27°C to 50°C, e.g. from about 30°C to 45°C, such as from 35°C to 40°C, for example from about 37°C to 40°C.

As the skilled man would appreciate, glass transition temperatures can be measured by differential scanning calorimetry (DSC) or rheology testing. In particular, glass transition temperature may be determined with DSC at a scan rate of 10°C/min in the first heating scan, wherein the glass transition is considered the mid-point of the change in enthalpy. A suitable instrument is a Perkin Elmer (Bucks, United Kingdom) DSC-7.

In other words, the formation of the scaffold is caused by exposing the polymer microparticles to a change in temperature, from a temperature that is below their Tg to a higher temperature. The higher temperature does not necessarily have to be equal to or above their Tg; any increase in temperature that is towards their Tg can trigger the required interaction between the polymer microparticles. In one embodiment, the formation of the scaffold is caused by exposing the polymer microparticles to a change in temperature, from a temperature that is below their Tg to a higher temperature, wherein the higher temperature is not more than 50°C below their Tg, such as not more than 30°C below their Tg or not more than 20°C below their Tg or not more than 10°C below their Tg.

In one embodiment, if the polymer microparticles are raised close to or above their Tg temperature on injection into the body, the polymer microparticles will crosslink/inter-link to one or more other polymer microparticles to form a scaffold. By cross-link/inter-link it is meant that adjacent polymer microparticles become joined together. For example, the polymer microparticles may cross-link/inter-link due to entanglement of the polymer chains at the surface of one polymer microparticles with polymer chains at the surface of another polymer microparticles. There may be adhesion, cohesion or fusion between adjacent polymer microparticles.

When the polymer microparticles come together and cross-link/inter-link, pores are formed in the resultant scaffold, as a consequence of the inevitable spaces between adjacent polymer microparticles. Such spaces/gaps between the polymer microparticles may not be filled with a hydrogel or other structural material. However, such spaces/gaps between the polymer microparticles may be filled with liquid carrier.

In one embodiment the scaffold material comprises discrete polymer microparticles which are capable of interacting to form a scaffold which have a Tg between about 35 °C and about 40°C, as well as other discrete polymer microparticles that have a Tg about 40°C. An agent for delivery may be incorporated into just one of the particle types or both. Preferably the agent for delivery is incorporated in at least the discrete particles that have a Tg above 40°C.

The scaffold may form without the generation of heat or loss of an organic solvent.

Formation of the scaffold from the scaffold material, once administered to a human or non-human animal, may take from about 20 seconds to about 24 hours, alternatively between about 1 minute and about 5 hours, alternatively between about 1 minute and about 1 hour, alternatively less than about 30 minutes, alternatively less than about 20 minutes. In one embodiment, the solidification occurs in between about 1 minute and about 20 minutes from administration.

The scaffold material may comprise from about 20% to about 80% polymer microparticles and from about 20% to about 80% carrier; from about 30% to about 70% polymer microparticles and from about 30% to about 70% carrier; e.g. the scaffold material may comprise from about 40% to about 60% polymer microparticles and from about 40% to about 60% carrier; the scaffold material may comprise about 50% polymer microparticles and about 50% carrier. The aforementioned percentages all refer to percentage by weight.

In one embodiment, the scaffold material can be used to form a scaffold that can resist a compressive load in excess of 2 MPa (thus is suitable for bone applications). The scaffold compressive strength may be a property of the scaffold in situ. Additionally, the scaffold compressive strength may be a property of the scaffold measured in vitro following sintering for at least 24 hours in a moist environment (for example 100% humidity) at about 37°C. In another embodiment, the scaffold may have a compressive strength of at least 0.5MPa after sintering for 2h in a moist environment (for example 100% humidity) at about 37°C.

Other aspects and embodiments of the description may not require a significant compressive strength, such as 2 MPa. For example, in an application where a film (i.e. a substantially thin film) of scaffold is desired, the level of the compressive strength of the scaffold may not be a relevant parameter. For example, in some applications a degree of flexibility of the scaffold may be desirable. Therefore, the present description also encompasses substantially flexible scaffold material. Such flexible scaffold material may be pliable, such that it does not crack, splinter or break when bent or folded. In one embodiment, the scaffold has a putty consistency. In one embodiment, the scaffold may maintain its flexibility following setting of the scaffold. Alternatively, the scaffold may be hard (for example not compressible or malleable by an average adult hand). In an embodiment wherein a film of scaffold is formed, the scaffold may be sufficiently flexible in order to roll it into a tube without fracturing.

The scaffold may be compressible without fracturing (for example a sponge consistency).

In one embodiment, the scaffold is formed ex situ (e.g. outside of the body/defect to be treated). In one embodiment, the scaffold material may be spread into a film, i.e. a substantially thin film prior to setting. The film may be formed by spreading the scaffold material onto a surface prior to setting. Spreading may comprise painting, rolling or injecting the scaffold material onto a surface to form a film of scaffold material. The forming of a film of scaffold may provide a flexible membrane of scaffold. In one embodiment, the film of scaffold may be 10mm or less in thickness. In another embodiment, the film of scaffold may be 8mm or less in thickness. In another embodiment, the film of scaffold may be 6mm or less in thickness. In another embodiment, the film of scaffold may be 5mm or less in thickness. In another embodiment, the film of scaffold may be between 2mm and 10mm in thickness.

In another embodiment, the film of scaffold may be less than 2mm in thickness. For example the film of scaffold may be between 100 microns and 2 mm in thickness. In another embodiment, the film of scaffold may be between 100 microns and 1 mm in thickness. In another embodiment, the film of scaffold may be between 150 microns and 1 mm in thickness. In another embodiment, the film of scaffold may be between 200 microns and 1 mm in thickness. In another embodiment, the film of scaffold may be between 500 microns and 1 mm in thickness. In embodiments where the thickness is less than 2mm, for example 100-500 microns, or 100 microns to 1mm, polymer particles of about 20-30 microns may be provided. Alternatively, polymer microparticles in the 20-100 micron size range can be used to form films of scaffold from 300microns to 1mm thick, or more. A film of scaffold may be formed which is at least as thick as the combined size of three polymer microparticles used to form the scaffold.

In methods wherein the scaffold material is spread into a film, the scaffold material may comprise smaller polymer microparticles, for example polymer microparticles may be 100 µm or less. In another embodiment, the polymer microparticles may be 50 µm or less. For example, the polymer microparticles may be between about 20 µm and about 100 µm, alternatively between about 20 µm and about 50 µm, alternatively between about 20 µm and about 30 µm. Additionally or alternatively, in methods wherein the scaffold material is spread into a film, the scaffold material may comprise a carrier to polymer microparticle ration of 1.2:1 or more. Additionally or alternatively, in methods wherein the scaffold material is spread into a film, the scaffold material may comprise a carrier to polymer microparticle ration of 1.5:1 or more. Additionally or alternatively, in methods wherein the scaffold material is spread into a film, the scaffold material may comprise a carrier to polymer microparticle ration of about 2:1. Additionally or alternatively, in methods wherein the scaffold material is spread into a film, the scaffold material may comprise a carrier to polymer microparticle ration of between about 1.2:1 and about 2:1.

### Polymer microparticles

The polymer microparticles may be provided dry, for example prior to mixing with any carrier. The polymer microparticles may be at least partially dispersible in the carrier. The polymer microparticles may not be soluble in the carrier at a temperature of 37°C or less.

The polymer microparticles may comprise or consist of one or more polymer. The polymer(s) may be synthetic polymer(s). The polymer microparticles may comprise one or more polymer selected from the group comprising poly (α-hydroxyacids) including poly (D,L-lactide-co-glycolide)(PLGA), poly D,L-lactic acid (PDLLA), polyethyleneimine (PEI), polylactic or polyglcolic acids, poly-lactide poly-glycolide copolymers, and poly-lactide poly-glycolide polyethylene glycol copolymers, polyethylene glycol (PEG), polyesters, poly (ε-caprolactone), poly (3-hydroxybutyrate), poly (s-caproic acid), poly (p-dioxanone), poly (propylene fumarate), poly (ortho esters), polyol/diketene acetals addition polymers, polyanhydrides, poly (sebacic anhydride) (PSA), poly (carboxybiscarboxyphenoxyphosphazene) (PCPP), poly [bis (p-carboxyphenoxy) methane] (PCPM), copolymers of SA, CPP and CPM (as described in Tamat and Langer in Journal of Biomaterials Science Polymer Edition, 3, 315-353. 1992 and by Domb in Chapter 8 of The Handbook of Biodegradable Polymers, Editors Domb A J and Wiseman R M, Harwood Academic Publishers), poly (amino acids), poly (pseudo amino acids), polyphosphazenes, derivatives of poly [(dichloro) phosphazene], poly [(organo) phosphazenes], polyphosphates, polyethylene glycol polypropylene block co-polymers for example that sold under the trade mark Pluronics^{™}, natural or synthetic polymers such as silk, elastin, chitin, chitosan, fibrin, fibrinogen, polysaccharides (including pectins), alginates, collagen, peptides, polypeptides or proteins, copolymers prepared from the monomers of any of these polymers, random blends of these polymers, any suitable polymer and mixtures or combinations thereof.

The polymer microparticles may comprise polymer selected from the group comprising poly(α-hydroxyacids) such as poly lactic acid (PLA), polyglycolic acid (PGA), poly(D,L-lactide-co-glycolide)(PLGA), poly D, L-lactic acid (PDLLA), poly-lactide poly-glycolide copolymers, and combinations thereof. In one embodiment, the polymer microparticles comprise PLGA.

The polymer microparticles may comprise polymer which is a blend of a poly(α-hydroxyacid) with poly(ethylene glycol) (PEG), such as a blend of a polymer or copolymer based on glycolic acid and/or lactic acid with PEG. In another embodiment, the polymer microparticles may not comprise PEG. In another embodiment, the polymer microparticles may be substantially free of PEG, for example, the polymer microparticles may comprise less than 2% PEG. In another embodiment, the polymer microparticles may comprise less than 1.5% PEG. In another embodiment, the polymer microparticles may comprise less than 1% PEG. In another embodiment, the polymer microparticles may comprise less than 0.5% PEG. In another embodiment, the polymer microparticles may comprise less than 0.2% PEG.

In one embodiment, the polymer microparticle comprises PLGA 95:5. Alternatively, the polymer microparticle may comprise PLGA 50:50. Alternatively, the polymer microparticle may comprise PLGA 85:15. Alternatively, the polymer microparticle may comprise any PLGA between PLGA 85:15 and PLGA 95:5. Alternatively, the polymer microparticle may comprise PLGA 65:35. Alternatively, the polymer microparticle may comprise PLGA 72:25. PLGA having monomer ratios between the above PLGA embodiments may also be considered.

In embodiments wherein PEG is provided as a plasticiser in the polymer microparticle, the PEG may be up to 10% of the polymer microparticle content. Alternatively, the PEG may be up to 8% of the polymer microparticle content. Alternatively, the PEG may be up to 6% of the polymer microparticle content. Alternatively, the PEG may be up to 3% of the polymer microparticle content. Alternatively, the PEG may be up to 2% of the polymer microparticle content. Alternatively, the PEG may be up to 1% of the polymer microparticle content. Alternatively, the PEG may be between 1 and 10% of the polymer microparticle content. Alternatively, the PEG may be between 5 and 8% of the polymer microparticle content. Alternatively, the PEG may be between 6 and 7% of the polymer microparticle content. Alternatively, the PEG may be between 2 and 6% of the polymer microparticle content. Alternatively, the PEG may be between 3 and 4% of the polymer microparticle content. Alternatively, the PEG may be about 6.5% of the polymer microparticle content.

In embodiments wherein PEG is provided as a plasticiser in the polymer microparticle, the PEG may have a molecular weight of 1000Da or less. Alternatively the PEG is 800Da or less. Alternatively the PEG is 600Da or less. In one embodiment, the PEG is PEG400.

The polymer microparticles may comprise a plasticiser, which may or may not be PEG. The plasticiser may comprise PLGA, such as low molecular weight PLGA, for example less than 10KDa PLGA. Additionally or alternatively, the plasticiser may comprise the monomers of PLGA (i.e. DL-lactide and/or glycolide).

The polymer microparticles may comprise a plasticiser selected from any of glycerine, polyethylene glycols, polyethylene glycol monomer ether, propylene glycol, sorbitol sorbitan solution, acetyl tributyl citrate, acetyl triethyl citrate, castor oil, diacetyl monoglycerides, dibutyl sebacate, diethyl phthalate, triacetin, tributyl citrate, triethyl citrate, or combinations thereof, optionally wherein the plasticisers are provided in an amount of 1-10% w/w.

The polymer microparticles may comprise a plasticiser selected from any of glycerine, polyethylene glycols, polyethylene glycol monomer ether, propylene glycol, sorbitol sorbitan solution, or combinations thereof, optionally wherein the plasticisers are provided in an amount of 1-10% w/w. The polymer microparticles may comprise a plasticiser selected from any of acetyl tributyl citrate, acetyl triethyl citrate, castor oil, diacetyl monoglycerides, dibutyl sebacate, diethyl phthalate, triacetin, tributyl citrate, triethyl citrate, or combinations thereof, optionally wherein the plasticisers are provided in an amount of 1-10% w/w.

The polymer microparticles may be biocompatible and/or biodegradable. By controlling the polymers used in the polymer microparticles the rate of scaffold degradation may be controlled.

The scaffold material may comprise one or more types of polymer microparticles made from one or more type of polymer. Furthermore, the scaffold material may comprise natural-polymer particles or non-polymer particles. The natural-polymer particles or non-polymer particles may be microparticles.

The non-polymer particles may comprise or consist of ceramic. The ceramic may comprise or consist of calcium sulphate (CS) or β-tricalcium phosphate (β-TCP). In another embodiment, the natural-polymer particles or non-polymer particles may comprise crystallised sugar molecules, such as crystallised particles of mannitol. Other sugar particles may be provided, such as glucose.

In one embodiment, the natural-polymer particles or non-polymer particles may comprise anti-oxidant. In one embodiment, the natural-polymer particles or non-polymer particles may comprise silica substituted ceramics. In one embodiment, the natural-polymer particles or non-polymer particles may comprise α-tricalcium phosphate. In one embodiment, the natural-polymer particles or non-polymer particles may comprise hydroxyapatite. In one embodiment, the natural-polymer particles or non-polymer particles may comprise calcium phosphate. Combinations of different natural-polymer particles or non-polymer particles may be considered.

The natural-polymer particles or non-polymer particles may be substantially similar or equal in size (according to an average particle size in a population) relative to the polymer microparticles. In another embodiment, the natural-polymer particles or non-polymer particles may be smaller in size (according to an average particle size in a population) relative to the polymer microparticles. For example, in one embodiment, the natural-polymer particles or non-polymer particles may be in powder form. A powder form may comprise particles of less than about 250 microns according to an average particle size in a population. In another embodiment, a powder form may comprise particles of less than about 150 microns according to an average particle size in a population. In another embodiment, a powder form may comprise particles of between about 20 and 250 microns according to an average particle size in a population.

In one embodiment, the natural-polymer or non-polymer particles are encapsulated within the polymer-microparticles. The encapsulation may be provided by the formation of the polymer microparticles in the presence of the natural-polymer or non-polymer particle, such as ceramic. For example, the encapsulation may occur through co-extrusion of the polymer for forming the polymer microparticles and the natural-polymer or non-polymer particles, such as ceramic. The non-polymer particle may be provided within the polymer microparticle according to the methods of the description herein.

The scaffold material may comprise between 1% and 55% natural-polymer or non-polymer particles, such as ceramic. In another embodiment, the scaffold material may comprise between 1% and 50% natural-polymer or non-polymer particles, such as ceramic. In another embodiment, the scaffold material may comprise between 1% and 55% natural-polymer or non-polymer particles, such as ceramic. In another embodiment, the scaffold material may comprise between 10% and 50% natural-polymer or non-polymer particles, such as ceramic. In another embodiment, the scaffold material may comprise between 20% and 50% natural-polymer or non-polymer particles, such as ceramic. In another embodiment, the scaffold material may comprise between 30% and 50% natural-polymer or non-polymer particles, such as ceramic. In another embodiment, the scaffold material may comprise between 40% and 50% natural-polymer or non-polymer particles, such as ceramic.

In an embodiment wherein the natural-polymer or non-polymer particles are encapsulated within the polymer microparticles the polymer microparticles may comprise between 1% and 55% (w/w) of natural-polymer or non-polymer particles, such as ceramic. Alternatively, in an embodiment wherein the natural-polymer or non-polymer particles are encapsulated within the polymer microparticles the polymer microparticles may comprise between 20% and 55% (w/w) of natural-polymer or non-polymer particles, such as ceramic. Alternatively, in an embodiment wherein the natural-polymer or non-polymer particles are encapsulated within the polymer microparticles the polymer microparticles may comprise between 20% and 50% (w/w) of natural-polymer or non-polymer particles, such as ceramic. Alternatively, in an embodiment wherein the natural-polymer or non-polymer particles are encapsulated within the polymer microparticles the polymer microparticles may comprise between 30% and 50% (w/w) of natural-polymer or non-polymer particles, such as ceramic. Alternatively, in an embodiment wherein the natural-polymer or non-polymer particles are encapsulated within the polymer microparticles the polymer microparticles may comprise between 40% and 50% (w/w) of natural-polymer or non-polymer particles, such as ceramic.

In an embodiment wherein natural-polymer or non-polymer particles, such as ceramic, are provided in the scaffold material, the scaffold material may comprise less than 40% v/v plasticiser in the carrier. In another embodiment wherein natural-polymer or non-polymer particles, such as ceramic, are provided in the scaffold material, the scaffold material may comprise less than 39% v/v plasticiser in the carrier. In another embodiment wherein natural-polymer or non-polymer particle, such as ceramic, are provided in the scaffold material, the scaffold material may comprise less than 35% v/v plasticiser in the carrier. In another embodiment wherein natural-polymer or non-polymer particle, such as ceramic, are provided in the scaffold material, the scaffold material may comprise less than 30% v/v plasticiser in the carrier. Alternatively, the plasticiser content may be less than 20%, 15%, 10% or 5% v/v of the carrier. In an embodiment wherein natural-polymer or non-polymer particles, such as ceramic, are provided in the scaffold material, the scaffold material may comprise about 1% v/v plasticiser in the carrier.

Where more than one type of polymer microparticle is used each polymer microparticle may have a different solidifying or setting property. For example, the polymer microparticles may be made from similar polymers but may have different gelling pHs or different melting temperatures or glass transition points.

In one embodiment, in order for the polymer particles to form a scaffold the temperature around the polymer microparticles, for example in the human or non human animal where the composition is administered, is approximately equal to, or greater than, the glass transition temperature of the polymer microparticles. At such temperatures the polymer microparticles may cross-link/inter-link to one or more other polymer microparticles to form a scaffold. By cross-link/inter-link it is meant that adjacent polymer particles become joined together. For example, the particles may cross-link/inter-link due to entanglement of the polymer chains at the surface of one polymer microparticle with polymer chains at the surface of another polymer microparticle. There may be adhesion, cohesion or fusion between adjacent polymer microparticles.

The scaffold material may comprise polymer microparticles which are formed of a polymer or a polymer blend that has a glass transition temperature (Tg) either close to or just above body temperature (such as from about 30°C to 45°C, e.g. from about 35°C to 40°C, for example from about 37°C to 40oC). Accordingly, at room temperature the polymer microparticles are below their Tg and behave as discrete polymer microparticles, but in the body the polymer microparticles soften and interact/stick to their neighbours. Preferably scaffold formation begins within 15 minutes of the raise in temperature from room to body temperature.

The polymer microparticles may be formed from a polymer which has a Tg from about 35°C to 40°C, for example from about 37°C to 40°C, wherein the polymer is a poly(α-hydroxyacid) (such as PLA, PGA, PLGA, or PDLLA or a combination thereof), or a blend thereof with poly(ethylene glycol) (PEG). At body temperature these polymer microparticles may interact to from a scaffold. The scaffold material may comprise only poly(α-hydroxyacid) /PEG particles or other particle types may be included.

The polymer microparticles may be formed from a blend of poly(D,L-lactide-coglycolide)(PLGA) and poly(ethylene glycol) (PEG) which has a Tg at or above body temperature. At body temperature these polymer microparticles can interact to from a scaffold, and during this process PEG may be lost from the surface of the polymer microparticles which will have the effect of raising the Tg and hardening the scaffold structure. The scaffold material may comprise only PLGA/PEG microparticles or other particle types may be included. In another embodiment, the scaffold material may comprise only PLGA microparticles. In another embodiment, the scaffold material, such as the polymer microparticles, may be substantially free of plasticiser, such as PEG.

Advantageously, providing a polymer microparticle which is substantially free of plasticiser, such as PEG, provides a leaner manufacturing process and improves the room temperature stability of the polymer microparticles. For example, due to the low glass transition temperatures of typical polymer microparticles, such as PLGA/PEG400 blends, they need to be stored in a fridge or freezer. In contrast a polymer microparticle which is substantially free of plasticiser would be capable of storage at room temperature. Such plasticiser free polymer microparticles may still be capable of setting into a scaffold with use of plasticisers in a carrier as described herein.

The scaffold material may comprise a mixture of temperature sensitive polymer microparticles and non-temperature sensitive particles. Preferably non temperature sensitive particles are particles with a glass transition temperature which is above the temperature at which the composition is intended to be used. In a composition comprising a mixture of temperature sensitive polymer microparticles and non-temperature sensitive particles the ratio of temperature sensitive polymer microparticles to non-temperature sensitive particles may be about 3:1, or lower, for example, 4:3. The temperature sensitive polymer microparticles may be capable of crosslinking or interlinking to each other when the temperature of the composition is raised to or above the glass transition a temperature of these polymer microparticles. By controlling the ratio of temperature sensitive polymer microparticles to non-temperature sensitive particles it may be possible to manipulate the porosity of the resulting scaffold.

In one embodiment, ceramic particles may additionally be present in the scaffold material. This will typically be a temperature insensitive particle type. Alternatively or additionally, polymer microparticles in the scaffold material may themselves contain a ceramic component. This will typically be a temperature insensitive particle type.

The inclusion of ceramic material either as separate particles or within the polymer microparticles may enhance osteoconductivity and/or add osteoinductivity.

The particles may be solid, that is with a solid outer surface, or they may be porous. The particles may be irregular or substantially spherical in shape.

The polymer microparticles may have a size in their longest dimension of between about 300 and about 500 µm. Polymer microparticles in the size range of 300-5000 µm may alternatively be called "polymer pellets". In another embodiment, the polymer microparticles may be 100 µm or less. In another embodiment, the polymer microparticles may be 50 µm or less. For example, the polymer microparticles may be between about 20 µm and about 100 µm, alternatively between about 20 µm and about 50 µm, alternatively between about 20 µm and about 30 µm. The size of the polymer particles may refer to the average size of a population of polymer microparticles.

The polymer microparticles may have a size in their longest dimension, or their diameter if they are substantially spherical, of less than about 3000µm and optionally more than about 1µm. In one embodiment, the particles have a size in their longest dimension, or their diameter, of less than about 1000µm. The polymer microparticles may have a size in their longest dimension, or their diameter, of between about 50µm and about 500µm, alternatively between about 100µm and about 500µm. Polymer microparticles of the desired size may be unable to pass through a sieve or filter with a pore size of about 50µm, but will pass through a sieve or filter with a pore size of about 500µm. Alternatively, polymer microparticles of the desired size may be unable to pass through a sieve or filter with a pore size of about 200µm, but will pass through a sieve or filter with a pore size of about 500µm.

The size of the polymer microparticles may be advantageously chosen by the skilled person for the intended application or type of scaffold required. For example, the use of pellet size (e.g. 300-5000 µm) polymer microparticles may be for increased porosity, where the gaps between the polymer microparticles may be larger relative to the use of smaller polymer microparticles. Such size control can provide control over the agent release rate, whereby a faster release rate may be provided by the choice of larger pellet size particles.

### The carrier

In one embodiment, the carrier is an aqueous carrier, such as water. The carrier may be an aqueous solution or suspension, such as saline, plasma, bone marrow aspirate, buffers, such as Hank's Buffered Salt Solution (HBSS), HEPES (4-(2-hydroxyethyl)-1-piperazineethanesulfonic acid), Ringers buffer, Krebs buffer, Dulbecco's PBS, or normal PBS; simulated body fluids, plasma platelet concentrate or tissue culture medium.

The carrier may, optionally, comprise one or more suspending agent. The suspending agent may be selected from carboxy methylcellulose (CMC), mannitol, polysorbate, poly propylene glycol, poly ethylene glycol, gelatine, albumin, alginate, hydroxyl propyl methyl cellulose (HPMC), hydroxyl ethyl methyl cellulose (HEMC), bentonite, tragacanth, dextrin, sesame oil, almond oil, sucrose, acacia gum and xanthan gum and combinations thereof. In one embodiment, the carrier comprises CMC.

The CMC may be provided in the carrier in an amount of 0.1% to 4% w/v. The CMC may be provided in the carrier in an amount of 0.1% to 3.5% w/v. The CMC may be provided in the carrier in an amount of 0.1% to 3% w/v. The CMC may be provided in the carrier in an amount of 0.1% to 2.5% w/v. The CMC may be provided in the carrier in an amount of 0.5% to 1% w/v.

The carrier may further comprise a polymer for enhancing the fluidity of the scaffold material. For example, the polymer may comprise Pluronic, such as Pluronic F127. The polymer, such as Pluronic F127, for enhancing the fluidity of the scaffold material may be provided in the carrier in an amount of about 1% w/v. The polymer, such as Pluronic F127, for enhancing the fluidity of the scaffold material may be provided in the carrier in an amount of about 0.5 to 2% w/v.

The carrier comprises the plasticisers. The plasticisers may be directly added to the carrier itself, for example, the plasticisers may not be provided in the carrier solely by diffusion from the polymer microparticles. In one embodiment, both the carrier and the polymer microparticles may comprise the plasticisers. In another embodiment, only the carrier and not the polymer microparticles may comprise the plasticisers. In another embodiment, only the polymer microparticles and not the carrier may comprise the plasticisers.

The plasticiser in the carrier may be selected from polyethylene glycol (PEG), polypropylene glycol, poly (lactic acid) or poly (glycolic acid) or a copolymer thereof, polycaprolactone, and low molecule weight oligomers of these polymers, or conventional plasticisers, such as, adipates, phosphates, phthalates, sabacates, azelates and citrates. The plasticiser may also be an alcohol such as ethanol or methanol. In one embodiment, the carrier may comprise ethanol. In one embodiment the plasticiser in the carrier does not comprise PEG. In another embodiment the plasticiser in the carrier comprises PEG.

Advantageously, providing the plasticisers in the carrier allows the polymer microparticles to be provided substantially plasticiser free, such as PEG free. As discussed above, this allows setting of the scaffold material into a solid scaffold in the absence of a plasticiser, such as PEG, in the polymer microparticles. Therefore, the polymer microparticles are easier and more economical to manufacture, and they can be stored at room temperature.

A first plasticizer may be provided in the carrier, wherein the first plasticiser is triethyl citrate, and second plasticiser may be provided in the carrier, wherein the second carrier comprises ethanol.

The first plasticiser may be provided in the carrier and the second plasticiser may be provided in the carrier and/or the polymer microparticle. The first plasticiser may be provided in the carrier and the second plasticiser may be PEG provided in the carrier and/or the polymer microparticle. The first plasticiser may be provided in the carrier and the second plasticiser may be PEG provided in the polymer microparticle.

The carrier may also include other known pharmaceutical excipients in order to improve the stability of the agent.

The carrier may comprise between 0.5% and 40% w/v plasticiser. Alternatively, the carrier may comprise between 0.5% and 30% w/v plasticiser. In another embodiment, the carrier may comprise between 0.5% and 20% w/v plasticiser. Alternatively, the carrier may comprise between 0.5% and 15% w/v plasticiser. The carrier may comprise between 0.5% and 10% w/v plasticiser. Alternatively, the carrier may comprise between 0.5% and 8% w/v plasticiser. Alternatively, the carrier may comprise between 0.5% and 6% w/v plasticiser. Alternatively, the carrier may comprise between 0.5% and 5% w/v plasticiser. Alternatively, the carrier may comprise between 1% and 6% w/v plasticiser. Alternatively, the carrier may comprise between 2% and 6% w/v plasticiser. Alternatively, the carrier may comprise about 0.5%, 0.79%, 1%, 2%, 3%, 4%, 5% or 6% w/v plasticiser.

In one embodiment, one or more additional excipient or delivery enhancing agent may also be included in the scaffold material, such as in the carrier, e.g. surfactants and/or hydrogels, in order to further influence release rate.

The carrier may interact with the polymer microparticles. The carrier may interact with the polymer microparticles to prevent or slow the formation of a scaffold and to allow the polymer microparticles to be administered to a human or non-human animal before a scaffold forms. The carrier may prevent interaction between the polymer microparticles due to separation of the particles by suspension in the carrier. It may be that the carrier completely prevents the formation of the scaffold prior to administration, or it may simply slow the formation, e.g. permitting the scaffold formation to begin but not complete formation prior to administration. In one embodiment the composition comprises sufficient carrier to prevent the formation of a scaffold even when the composition is at a temperature, which, in the absence of the carrier, would cause the polymer microparticles to form a scaffold. In one embodiment, the scaffold material comprises sufficient carrier to slow the formation of a scaffold such that when the scaffold material is at a temperature which, in the absence of the carrier, would cause the polymer microparticles to readily form a scaffold, a scaffold does not readily form, e.g. does not form over a timescale such as one hour to five hours.

The carrier may interact with the polymer microparticles and cause the surface of the polymer microparticles to swell, whilst remaining as discrete polymer microparticles, thus allowing administration by injection. However, once the composition has been administered and the carrier begins to dissipate the polymer microparticles may begin to de-swell. De-swelling may assist the joining together of polymer microparticles.

Interaction of the polymer microparticles with the carrier may cause the glass transition temperature of the polymer microparticles to change. For example, the interaction may cause the glass transition temperature to be lowered. Interaction of the polymer microparticles with the carrier may cause the glass transition temperature of the polymer microparticle surface to change. For example, the interaction may cause the glass transition temperature of the surface of the polymer microparticles to be lowered.

The carrier may act as a lubricant to allow the polymer microparticles to be administered to a human or non-human animal, for example by injection. The carrier may provide lubrication when the scaffold material is dispensed from a syringe. The carrier may help to reduce or prevent shear damage to polymer microparticles dispensed from a syringe.

The ratio of carrier to polymer microparticles in the scaffold material may be at least 1:1. The ratio of carrier to polymer microparticles in the scaffold material may be at least 1.5:1. The ratio of carrier to polymer microparticles in the scaffold material may be at least 1.2:1. In one embodiment, the ratio of carrier to polymer microparticles in the scaffold material may be between 0.7:1 and 2:1.

The carrier may further comprise a buffer. For example plasticisers such as TEC and TA can be acidic and a buffer may be provided to reduce the acidity of such components. Any suitable buffer may be provided, for example PBS, Tris buffer, or sodium bicarbonate.

### The Agent

With reference to a power form of the agent or powdered agent, the powder may be dry powder. For example the dry powder may have substantially no water content. Alternatively the term dry may be a water activity of less than 0.5Aw, or less than less than 0.3Aw, or less than 0.1Aw.

The powdered agent may be in crystalline, semi-crystalline or amorphous form. In one embodiment, the powdered agent may be in crystalline form.

In one embodiment, the powdered agent is encapsulated amongst the scaffold of polymer microparticles and additional agent may be encapsulated within the polymer microparticles. The additional agent may be in any form, for example a liquid form, such as a solution or suspension, a paste, a gel, or a powder form. The additional agent may be a different agent relative to the powder agent. Alternatively, the additional agent may be the same agent as the powder agent, but in a different form such as a solution or suspension, a gel, or a paste (i.e. the additional agent may not be in a powder form).. Reference to the form of the agent, such as powder form, liquid, paste or gel form may refer to the condition of the agent at the point of addition to the mixture or blend (i.e. it is not intended to refer to the form of the agent following use, for example in situ after scaffold formation).

The additional agent may be provided in the polymer microparticles during the formation of the polymer microparticles, for example by adding to the polymer for extrusion into polymer microparticles.

In one embodiment, the agent is only provided as a powdered agent to be encapsulated amongst the scaffold of polymer microparticles. For example, no other agent, or form of agent, is provided, for example, in the polymer microparticles.

Other embodiments of the description herein may be practised with the provision of an agent in the scaffold material for release of the agent, but the agent may not be required to be added in a powder form. Therefore, some embodiments of the description may provide an agent in the scaffold material in a non-powder form. For example the agent may be solubilised in the carrier. Additionally or alternatively, the agent may be provided/encapsulated in the polymer microparticles. In another embodiment, the agent may be provided to the scaffold material as a separate liquid phase relative to the carrier.

The agent may be a therapeutically, prophylactically or diagnostically active substance. It may be any bioactive agent.

In another embodiment, the powdered agent may be a non-therapeutic agent, for example a protective agent or a second agent provided to augment or protect a first powdered agent that may be therapeutically, prophylactically or diagnostically active substance. In one embodiment a second powdered agent may be provided to enhance the stability of function of a first powdered agent. The powdered agent may comprise cyclodextrin.

In one embodiment the powdered agent may comprise carboxymethyl cellulose (CMC). The provision of powdered CMC may be provided to alter the scaffold setting properties.

The agent for delivery may be a drug, a cell, signalling molecule, such as a growth factor, or any other suitable agent. For example, the agent may comprise amino acids, peptides, proteins, sugars, antibodies, nucleic acid, antibiotics, antimycotics, growth factors, nutrients, enzymes, hormones, steroids, synthetic material, adhesion molecules, colourants/dyes (which may be used for identification), radioisotopes (which may be for X-ray detection and/or monitoring of degradation), and other suitable constituents, or combinations thereof.

Other agents which may be added include but are not limited to epidermal growth factor, platelet derived growth factor, basic fibroblast growth factor, vascular endothelial growth factor, insulin-like growth factor, nerve growth factor, hepatocyte growth factor, transforming growth factors and other bone morphogenic proteins, cytokines including interferons, interleukins, monocyte chemotactic protein-1 (MCP-1), oestrogen, testosterone, kinases, chemokinases, glucose or other sugars, amino acids, calcification factors, dopamine, amine-rich oligopeptides, such as heparin binding domains found in adhesion proteins such as fibronectin and laminin, other amines, tamoxifen, cis-platin, peptides and certain toxoids. Additionally, drugs (including statins and NSAIDs), hormones, enzymes, nutrients or other therapeutic agents or factors or mixtures thereof may be included.

The agent may comprise nucleic acid, such as DNA, RNA, or plasmid.

In some embodiments, the agent for delivery is a statin, e.g. simvastatin, atorvastatin, fluvastatin, pravastatin or rosuvastatin. The statin may be simvastatin. Embodiments in which the agent is a statin are particularly suitable for the treatment of orthopaedic indications, craniomaxillofacial surgery and dentistry.

In an embodiment wherein an agent is part of (i.e. encapsulated within) the polymer microparticle, the agent may be up to 50% of the content of the microparticle. In another embodiment wherein an agent is part of (i.e. encapsulated within) the polymer microparticle, the agent may be up to 40% of the content of the microparticle. In another embodiment wherein an agent is part of (i.e. encapsulated within) the polymer microparticle, the agent may be up to 30% of the content of the microparticle. In another embodiment wherein an agent is part of (i.e. encapsulated within) the polymer microparticle, the agent may be up to 20% of the content of the microparticle. In another embodiment wherein an agent is part of (i.e. encapsulated within) the polymer microparticle, the agent may be up to 10% of the content of the microparticle. In another embodiment wherein an agent is part of (i.e. encapsulated within) the polymer microparticle, the agent may be between 10% and 50% of the content of the microparticle. In another embodiment wherein an agent is part of (i.e. encapsulated within) the polymer microparticles, the agent may be between 1% and 50% of the content of the polymer microparticles. In another embodiment wherein an agent is part of (i.e. encapsulated within) the polymer microparticles, the agent may be between 0.1% and 50% of the content of the polymer microparticles. In another embodiment wherein an agent is part of (i.e. encapsulated within) the polymer microparticles, the agent may be between 0.5% and 50% of the content of the polymer microparticles. In another embodiment wherein an agent is part of (i.e. encapsulated within) the polymer microparticles, the agent may be between 0.1% and 1% of the content of the polymer microparticles. In another embodiment wherein an agent is part of (i.e. encapsulated within) the polymer microparticles, the agent may be between 0.5% and 10% of the content of the polymer microparticles. In another embodiment wherein an agent is part of (i.e. encapsulated within) the polymer microparticles, the agent may be between 0.1% and 20% of the content of the polymer microparticles. The percentage may be w/w.

In an embodiment wherein an agent is provided in the carrier, the agent may be up to 75% of the content of the carrier. In another embodiment wherein an agent is provided in the carrier, the agent may be up to 60% of the content of the carrier. In another embodiment wherein an agent is provided in the carrier, the agent may be up to 50% of the content of the carrier. In another embodiment wherein an agent is provided in the carrier, the agent may be up to 40% of the content of the carrier. In another embodiment wherein an agent is provided in the carrier, the agent may be up to 30% of the content of the carrier. In another embodiment wherein an agent is provided in the carrier, the agent may be up to 20% of the content of the carrier. In another embodiment wherein an agent is provided in the carrier, the agent may be up to 10% of the content of the carrier. In another embodiment wherein an agent is provided in the carrier, the agent may be between 10% and 75% of the content of the carrier, or between 20% and 50% of the content of the carrier. The percentage may be w/v.

In an embodiment wherein an agent is in a powder form and mixed with the polymer microparticle prior to setting, the agent may be up to 75% of the content of the scaffold material. In another embodiment wherein an agent is in a powder form and mixed with the polymer microparticle prior to setting, the agent may be up to 60% of the content of the scaffold material. In another embodiment wherein an agent is in a powder form and mixed with the polymer microparticle prior to setting, the agent may be up to 50% of the content of the scaffold material. In another embodiment wherein an agent is in a powder form and mixed with the polymer microparticle prior to setting, the agent may be up to 40% of the content of the scaffold material. In another embodiment wherein an agent is in a powder form and mixed with the polymer microparticle prior to setting, the agent may be up to 30% of the content of the scaffold material. In another embodiment wherein an agent is in a powder form and mixed with the polymer microparticle prior to setting, the agent may be up to 20% of the content of the scaffold material. In another embodiment wherein an agent is in a powder form and mixed with the polymer microparticle prior to setting, the agent may be between 10% and 75% of the content of the scaffold material, or between 20% and 50% of the content of the scaffold material, alternatively between 20% and 30% of the content of the scaffold material.

The agent release may be controlled, that is, not all of the agent may be released in one large dose. The scaffold produced may permit the kinetics of agent release from the carrier to be controlled. The rate of release may be controlled by controlling the size and/or number of the pores in the scaffold and/or the rate of degradation of the scaffold. Other factors that can be controlled are the concentration of any suspending agent included in the carrier, the viscosity or physiochemical properties of the composition, and the choice of carrier.

The agent may be released by one or more of: diffusion of the agent through the pores; degradation of the scaffold leading to increased porosity and improved outflow of fluid carrying the agent; and physical release of agent from the polymer microparticles. It is within the abilities of the skilled person to appreciate that the size and/or number of the pores in the scaffold and/or the rate of degradation of the scaffold can readily be selected by appropriate choice of starting material so as to achieve the desired rate of release.

Diffusion of the agent away from the scaffold can occurs due to diffusion driven by a concentration gradient and the natural flow of body fluids through and away from the scaffold.

The agent may be released in an amount effective to have a desired local or systemic physiological or pharmacologically effect.

The scaffold may allow for agent release to be sustained for some time, for example at least about 2 hours, at least about 4 hours, at least about 6 hours, at least about 10 hours, at least about 12 hours, or at least about 24 hours. In one embodiment, the sustained release may be over at least 48 hours. In another embodiment, the sustained release may be over at least a week. In another embodiment, the sustained release may be over at least a 10 days.

Delivery of an agent means that the agent may be released from the scaffold into the environment around the scaffold, for example surrounding tissues.

The formed scaffold may allow a substantially zero or first order release rate of the agent from the scaffold. A zero order release rate is a constant release of the agent over a defined time. A first order release rate may also be considered a "burst release".

In one embodiment, the initial day 1 burst release is less than about 25-33% of total loading (such as less than about 20% or more such as less than about 10%, alternatively, less than about 5%). This initial burst is may then be followed by 1-2% release per day for about 14 days (which may equate to about 0.5-2mcg/day). Release of drug may continue for at least 14 days. Release of drug may continue for at least 20 days, 30 days, 40 day or 50 days. In some embodiments, release continues for about 14 to 56 days. In some embodiments release continues for more than 56 days.

In other embodiments, release kinetics can be modified by the use of mixed molecular weight PLGA polymers, which can effectively increase either the initial or longer-term release and help to avoid any therapeutic lag phase (European Journal of Pharmaceutics and Biopharmaceutics Volume 50, Issue 2, September 2000, Pages 263-270).

In other embodiments other release modifiers may be used to adjust release kinetics. For example, adjustments to the viscosity of a carboxymethycellulose-containing liquid phase residing within the scaffold pores may be made.

It is possible to use any animal cell with the scaffold material of the description. Examples of cells which may be used include bone, osteoprogenitor cells, cartilage, muscle, liver, kidney, skin, endothelial, gut, intestinal, cardiovascular, cardiomycotes, chondrocyte, pulmonary, placental, amnionic, chorionic, foetal or stem cells. Where stem cells are used, preferably non-embryonic stem cells are used. The cells may be included for delivery to the site of scaffold formation, or they may be included and intended to be retained in the scaffold, for example, to encourage colonisation of the scaffold.

In one embodiment, viable cells are provided in the scaffold material, for example, prior to formation/setting of the scaffold. For example, viable cells may be added to the scaffold material prior to setting.

In one embodiment, the surface of the polymer microparticles may be treated prior to introducing cells in order to enhance cell attachment. Surface treatments may comprise coating techniques to coat the surfaces of the polymer microparticles with an agent capable of enhancing or facilitating cell attachment. Additionally or alternatively, surface treatments may comprise physical or chemical modifications to the surface of the polymer microparticles. In surface coating, the polymer microparticles can be coated with materials that change their biological interactions, by altering surface charge, hydrophilicity and/or receptor-binding moieties. Such examples include, but are not limited to, chemical plasmas, peptides or carbohydrates, extracellular matrix components such as fibronectin or vitronectin or fragments thereof, poly-L-ornithine, polylysine and/or polyallylamines. In one embodiment, in surface physical/chemical modification, the polymer microparticle surfaces can modified by treating them with alkaline solutions such as NaOH solutions. In one embodiment, in surface physical/chemical modification, the polymer microparticle surfaces can be made rougher by treating them with alcohols, acids or bases. In another embodiment, in surface physical/chemical modification, the polymer microparticle surfaces can be made more hydrophilic and rougher by treating them with hydro-alcoholic alkaline solutions.

### Setting

In one embodiment, the setting of the scaffold material to form the scaffold is *in situ.* For example, the setting may take place post-administration, for example within a bone defect. Alternatively, setting may be provided ex situ, for example to provide a scaffold outside of the body. In one embodiment, the setting of the scaffold material to form the scaffold is at about 37 °C. In one embodiment, the setting of the scaffold material to form the scaffold is at about 35 °C or less. The setting of the scaffold material to form the scaffold may be in a humid environment, for example 100% humidity, alternatively at least 90% humidity. The setting of the scaffold material to form the scaffold may be whilst submerged in a solution. Reference to setting herein may also refer to sintering.

### Other Aspects

Described is a method of forming a scaffold material, the method comprising:
providing polymer microparticles;
suspending the polymer microparticles in a liquid carrier to form a scaffold material, which is a polymer microparticle suspension, wherein the liquid carrier comprises the claimed plasticisers; and
optionally setting the polymer microparticle suspension such that it sets into a solid scaffold of polymer microparticles.

Advantageously the methods of the description herein allow the skilled person to select appropriate scaffold properties or setting properties, for example when a plasticiser, such us TEC, is used in the liquid carrier it is possible to sinter particles made by PLGA/ceramic blends within 15 minutes to form a scaffold. Further advantageously, very low concentrations of plasticiser may be used according to the methods of the description. By choosing the concentration of plasticiser, such as TEC, it is possible to control the setting properties of the scaffold material.

In one embodiment, the plasticiser in the carrier is the first plasticiser and the second plasticiser is provided in the carrier and/or polymer microparticle.

In one embodiment, the polymer microparticle may not comprise PEG. The polymer microparticle may be substantially PEG free. In one embodiment, the polymer microparticles may comprise less than 0.5% PEG, or less than 0.2% PEG, or less than 0.1% PEG.

Described is a method of forming a scaffold material, the method comprising:
providing polymer microparticles;
suspending the polymer microparticles in a liquid carrier to form a scaffold material, which is a polymer microparticle suspension, wherein the scaffold material comprises a first plasticiser in the polymer microparticles and/or the liquid carrier, and a second plasticiser in the liquid carrier,
wherein, the first plasticiser is selected from any one of TEC (triethyl citrate), alcohol,
benzoic acid, triacetin, NMP, DMSO and PEG; and the second plasticiser is TEC (triethyl citrate) or triacetin (TA), wherein the first and second plasticisers are different; and
optionally setting the polymer microparticle suspension such that it sets into a solid scaffold of polymer microparticles.

In one embodiment, the first plasticizer is triethyl citrate, and the second plasticiser is ethanol. In another embodiment, the first plasticizer is triacetin, and the second plasticiser is ethanol. In one embodiment, the first plasticizer is triethyl citrate or triacetin, and the second plasticiser is PEG in the polymer microparticle.

In one embodiment, the second plasticiser comprises TEC (triethyl citrate) and the first plasticiser is selected from any one of PEG, DMSO, NMP, ethanol, benzoic acid, and triacetin (TA).

In another
embodiment, the second plasticiser comprises triacetin and the first plasticiser is selected from any one of PEG, DMSO, NMP, TEC (triethyl citrate), ethanol, and benzoic acid.

In an embodiment wherein a first and second plasticiser is provided, the polymer microparticle may not comprise PEG. In an embodiment wherein a first and second plasticiser is provided, the polymer microparticles may be substantially PEG free. In another embodiment wherein a first and second plasticiser is provided, the polymer microparticles may comprise less than 0.5% w/w PEG, or less than 0.2% w/w PEG, or less than 0.1% w/w PEG.

Providing the two or more plasticisers according to the description allows greater setting control of the scaffold material into a solid scaffold. For example the ratio of carrier to polymer microparticles in the scaffold material may be increased without also inadvertently prolonging the scaffold setting time. Therefore, the present description allows high carrier to polymer microparticle ratio. In one embodiment, the carrier to polymer microparticle ratio is at least 0.7:1 v/w. In another embodiment, the carrier to polymer microparticle ratio is at least 1:1 v/w. In another embodiment, the carrier to polymer microparticle ratio is at least 1.2:1 v/w. In another embodiment, the carrier to polymer microparticle ratio is at least 1.5:1 v/w. In another embodiment, the carrier to polymer microparticle ratio is at least 1.8:1 v/w. In another embodiment, the carrier to polymer microparticle ratio is at least 2:1 v/w. In another embodiment, the carrier to polymer microparticle ratio is between about 1.2:1 v/w and about 2:1 v/w. Advantageously, the high carrier to polymer microparticle ratio of the description can allow lower viscosity scaffold material to be provided without prolonging the setting times. The higher carrier to polymer particle ratio achievable in the present description allows the scaffold material to be more fluidic or malleable prior to setting. Advantageously, the scaffold material can be easier to inject prior to setting due to a lower viscosity of the scaffold material. Furthermore, the scaffold material may be more formable to a shape, such as a bone defect to be repaired. A higher carrier to polymer microparticle ratio also aids the forming of thin films or membranes of the scaffold material for applications where a thin membrane/film scaffold layer is required. Therefore, the low viscosity scaffold material may be spread into a film prior to setting into a scaffold.

Described is a method of forming a scaffold material comprising a natural-polymer or non-polymer particle content, the method comprising:
blending a polymer with natural-polymer or non-polymer particles;
forming polymer microparticles from the blend, wherein the polymer particles have the natural-polymer or non-polymer particles encapsulated therein; and
suspending the polymer microparticles in the liquid carrier to form a polymer microparticle suspension; and
further optionally setting the polymer microparticle suspension such that it sets into a solid scaffold of polymer microparticles.

Encapsulation of the natural-polymer or non-polymer particles in the polymer of the polymer microparticles is understood to include the polymer being dispersed amongst and surrounding the natural-polymer or non-polymer particles (e.g. not just a polymer surface coating on the natural-polymer or non-polymer particles). For example, the polymer microparticles may comprise natural-polymer or non-polymer particles entirely encased within the polymer and natural-polymer or non-polymer particles exposed at the surface of the polymer microparticles. For example, the polymer microparticles may be discreet particles having a plurality of natural-polymer particles or non-polymer particles encapsulated therein.

In one embodiment non-polymer particles, such as ceramic particles, are provided.

In one embodiment, blending the polymer with natural-polymer or non-polymer particles may comprise the step of dry mixing the polymer with natural-polymer or non-polymer particles. The dry mixture of the polymer and natural-polymer or non-polymer particles may be hot-melt extruded and the extrudate may be pelleted to form polymer microparticles having natural-polymer or non-polymer particles encapsulated therein.

The dry mixture of the polymer and natural-polymer particles may be blended together by physically mixing them. The dry mixture of the polymer and natural-polymer or non-polymer particles may be blended together by mixing them in a solution and spray drying. The dry mixture of the polymer and natural-polymer or non-polymer particles may be blended together by spray coating the polymer onto the natural-polymer or non-polymer particles. The dry mixture of the polymer and natural-polymer or non-polymer particles may be blended together by combining them into an organic solvent and forming an emulsion with an inorganic phase. The dry mixture of the polymer and natural-polymer or non-polymer particles may be blended together by combining them in an organic solvent and prilling.

The polymer microparticles comprising encapsulated natural-polymer or non-polymer particles may be between about 300 and about 400 microns in size as an average, as measured across their longest dimension. The natural-polymer or non-polymer particles may be substantially equal or similar in size to the polymer microparticles, for example the natural-polymer or non-polymer particles may be between about 300 and about 400 microns in size as an average, as measured across their longest dimension. Alternatively, the natural-polymer or non-polymer particles may be between about 20 and about 500 microns in size as an average, as measured across their longest dimension.

When smaller particles are required, the further step of cryomilling may be provided. Therefore, the method may further comprise cryomilling the polymer microparticles, to form smaller polymer microparticles. The smaller polymer microparticles may be 100 µm or less. In another embodiment, the smaller polymer microparticles may be 50 µm or less. For example, the smaller polymer microparticles may be between about 20 µm and about 100 µm, alternatively between about 20 µm and about 50 µm, alternatively between about 20 µm and about 30 µm. The size of the polymer particles may refer to the average size of a population of polymer microparticles.

The scaffold material may comprise between 1% and 55% natural-polymer or non-polymer particles, such as ceramic. In another embodiment, the scaffold material may comprise between 1% and 50% natural-polymer or non-polymer particles, such as ceramic. In another embodiment, the scaffold material may comprise between 1% and 55% natural-polymer or non-polymer particles, such as ceramic. In another embodiment, the scaffold material may comprise between 10% and 50% natural-polymer or non-polymer particles, such as ceramic. In another embodiment, the scaffold material may comprise between 20% and 50% natural-polymer or non-polymer particles, such as ceramic. In another embodiment, the scaffold material may comprise between 30% and 50% natural-polymer or non-polymer particles, such as ceramic. In another embodiment, the scaffold material may comprise between 40% and 50% natural-polymer or non-polymer particles, such as ceramic. The percentage may be w/w.

In one embodiment, the polymer microparticles may comprise between 1% and 55% (w/w) of natural-polymer or non-polymer particles, such as ceramic. Alternatively, the polymer microparticles may comprise between 20% and 55% (w/w) of natural-polymer or non-polymer particles, such as ceramic. Alternatively, the polymer microparticles may comprise between 20% and 50% (w/w) of natural-polymer or non-polymer particles, such as ceramic. Alternatively, the polymer microparticles may comprise between 30% and 50% (w/w) of natural-polymer or non-polymer particles, such as ceramic. Alternatively, the polymer microparticles may comprise between 40% and 50% (w/w) of natural-polymer or non-polymer particles, such as ceramic.

The scaffold material comprising natural-polymer or non-polymer particles, such as ceramic, may comprise less than 40% w/v plasticiser in the carrier. In another embodiment, the scaffold material comprising natural-polymer or non-polymer particles, such as ceramic, may comprise less than 38% w/v plasticiser in the carrier. In another embodiment, the scaffold material comprising natural-polymer or non-polymer particles, such as ceramic, may comprise less than 35% w/v plasticiser in the carrier. In another embodiment, the scaffold material comprising natural-polymer or non-polymer particles, such as ceramic, may comprise less than 30% w/v plasticiser in the carrier. Alternatively, the plasticiser content may be less than 20%, 15%, 10% or 5% w/v in the carrier. The scaffold material comprising natural-polymer or non-polymer particles, such as ceramic, may comprise about 1% w/v plasticiser in the carrier.

The natural-polymer particles or non-polymer particles may be microparticles. The non-polymer particles may comprise or consist of ceramic. The ceramic may comprise or consist of calcium sulphate (CS) or β-tricalcium phosphate (β-TCP). In another embodiment, the natural-polymer particles or non-polymer particles may comprise crystallised sugar molecules, such as crystallised particles of mannitol. Other sugar particles may be provided, such as glucose. In one embodiment, the natural-polymer particles or non-polymer particles may comprise anti-oxidant.

The plasticiser may comprise PEG. The mixture for hot-melt extrusion may comprise PEG.

Both natural-polymer particles and non-polymer particles may be provided for encapsulation with the polymer into the polymer microparticles.

The polymer for blending with the natural-polymer or non-polymer particles may comprise at least 30% of the mixture. In another embodiment, the polymer for blending with the natural-polymer or non-polymer particles may comprise at least 40% of the mixture. In another embodiment, the polymer for blending with the natural-polymer or non-polymer particles may comprise at least 45% of the mixture.

In another embodiment, the polymer for blending with the natural-polymer or non-polymer particles may comprise at least 48% or 49% of the mixture. In another embodiment, the polymer for blending with the natural-polymer or non-polymer particles may comprise at least 50% of the mixture. In another embodiment, the polymer for blending with the natural-polymer or non-polymer particles may comprise at least 60%, 70% or 80% of the mixture. In another embodiment, the polymer for blending with the natural-polymer or non-polymer particle may comprise at least 90% of the mixture.

In one embodiment, the polymer microparticles may comprise between about 10% and about 50% of natural-polymer or non-polymer particles; between about 40% and 85% polymer; and between about 1% and about 10% plasticiser, wherein the total amounts do not exceed 100%.

### METHOD/SYSTEM OF MODIFYING SCAFFOLD FORMING PROPERTIES

Advantageously, the use of plasticiser in the carrier at a range of concentrations can provide control over the scaffold setting properties of a scaffold material according to the description, such that a preferred setting temperature or a preferred setting time can be achieved.

Described is a method of forming a scaffold material which is capable of setting in less than 5 minutes, wherein the scaffold material is provided in accordance with any of the methods of the description herein, and wherein the plasticiser is provided in the carrier in a range of between about 4% and about 6% w/v.

Described is a method of forming a scaffold material having a scaffold setting time of between about 5 and about 15 minutes, wherein the scaffold material is provided in accordance with any of the methods of the description herein, and wherein the plasticiser is provided in the carrier in a range of between about 2.5% and about 3.5% w/v.

Described is a method of forming a scaffold material suitable for forming a scaffold having a 1^{st} order agent release kinetic, wherein the scaffold material is provided in accordance with methods of the description herein, and wherein the agent is provided as a powder prior to blending with polymer to form the polymer microparticles of the scaffold material.

Methods of forming the scaffold according to description herein may comprise the step of setting the scaffold by administrating/applying the scaffold material to a site for tissue repair or replacement. The site for a tissue repair or replacement may be a tissue *in situ,* in a body of a patient, or in a tissue in vitro/ex situ. The application may by methods described herein, such as implantation, injection, or moulding into the site for repair or replacement.

### COMPOSITION - scaffold material pre-scaffold formation

Described is a scaffold material produced by any method of the description.

In one embodiment, the scaffold or scaffold material may be suitable for bone repair. Described is scaffold material for forming a scaffold, wherein the scaffold material comprises:
polymer microparticles;
a liquid carrier suspending the polymer microparticles, wherein the liquid carrier comprises a plasticiser; and a second plasticiser provided in the carrier and/or the polymer microparticles.

### SCAFFOLD (post-formation)

Described is a scaffold produced by any method of the description.

Described is a scaffold for controlled release of an agent, wherein the scaffold comprises:
cross-linked/inter-linked polymer microparticles; and
an agent, wherein the agent is in a powder form and is encapsulated amongst and between the polymer microparticles.

Described is a scaffold for bone repair, wherein the scaffold comprises:
cross-linked/inter-linked polymer microparticles; and
natural-polymer particles and/or non-polymer particles (such as ceramic), wherein the natural-polymer particles and/or non-polymer particles are encapsulated within the polymer microparticles.

Described is a method of delivering an agent to a subject comprising providing a scaffold material, wherein the agent is located within polymer microparticles within the scaffold material; administering the scaffold material to a subject; allowing the scaffold material to solidify/self-assemble in the subject to form a scaffold; and allowing the agent contained within the scaffold material to be released into the subject at the site of administration.

Described is a method of delivering an agent to a subject comprising providing a scaffold material, wherein the agent is located amongst the polymer microparticles within the scaffold material; administering the scaffold material to a subject; allowing the scaffold material to solidify/self-assemble in the subject to form a scaffold; and allowing the agent contained within the scaffold material to be released into the subject at the site of administration.

The method may be practised on tissue in vivo or in vitro.

The agent (encapsulated within polymer microparticles) may optionally be added to the scaffold material immediately prior to administration to the subject.

In one embodiment, in step d) the agent release is sustained over a period at least 12 hours.

The scaffold material or scaffold may be for use in a method of treatment or prevention of a condition selected from: neurodegeneration disorders (e.g. post stroke, Huntington's, Alzheimer's disease, Parkinson's disease), bone-related disorders (including osteoarthritis, spinal disk atrophy, bone cavities requiring filling, bone fractures requiring regeneration or repair), burns, cancers, liver disorders (including hepatic atrophy), kidney disorders (including atrophy of the kidney), disorders of the bladder, ureter or urethra (including damaged ureter or damaged bladder requiring reconstruction, prolapse of the bladder or the uterus), diabetes mellitus, infertility requiring IVF treatment, muscle wasting disorders (including muscular dystrophy), cardiac disorders (e.g. damaged cardiac tissue post myocardial infarction, congestive heart disease), eye disorders (e.g. damaged or diseased cornea), damaged vasculature requiring regeneration or repair, ulcers, and damaged tissue requiring regeneration or reconstruction (including damaged organ requiring regeneration or reconstruction, and damaged nerves requiring regeneration or reconstruction).

In some embodiments the treatment is dental bone repair, such as dental ridge restoration. In other embodiments the treatment is the repair of non-union fractures. In other embodiments the treatment is spinal fusion.

Dental bone graft substitutes are primarily used in implant procedures requiring additional bone support. Bone regeneration is enhanced with advanced products, allowing dental bone grafting procedures to be performed on patients who would otherwise not be able to receive such treatment. In approximately 40% of all dental implant cases, there is not enough bone to ensure proper implant integration, and bone graft substitutes are required. Tooth extraction can result in deterioration of alveolar bone, resulting in a chronic progressive condition termed residual ridge resorption (RRR). Standard bone grafting options result in secondary lesions, immunologic rejection and poor long-term outcomes. Osteoinductive factors released from a non-immunogenic delivery system could provide an answer.

Grafting techniques are making it possible to expand the candidate pool for implants to include a sizable population of edentulous patients who were poor candidates for dental implantation due to severe bone resorption.

Treatments that positively influence bone healing following fracture, and subsequently shorten the time necessary for bone union are of great interest. Surgical intervention in non-unions is required to re-expose living tissue and to insert an osteoinductive graft material. Using autograft or allograft material, this treatment is successful in 70-80% of cases and costs an estimated $14,000 per patient. There is therefore much interest in more effective graft materials.

Spinal fusion is used to surgically treat vertebral abnormalities such as spinal curvatures (scoliosis or kyphosis), slipped discs (following discectomy), or fractures. The procedure uses graft materials (with or without pedicle screws, plates or cages) or other devices to fuse vertebrae together. Many patients complain of donor site pain from the autograft harvest for up to 2 years postoperatively. These complications have driven the search for and subsequent use of alternatives. The description provides such alternatives in the form of the systems, compositions and methods described herein.

The scaffold or scaffold material formed by any method and/or composition of the description may be used to treat damaged tissue. In particular, the scaffold or scaffold material may be used to encourage or allow cells to re-grow in a damaged tissue. The invention may therefore be used in the treatment of tissue damage, including in the regeneration or reconstruction of damaged tissue.

The scaffold material of the description may be used to produce scaffolds for use in the treatment of a disease or medical condition, such as, but not limited to, Alzheimer's disease, Parkinson's disease, osteoarthritis, burns, spinal disk atrophy, cancers, hepatic atrophy and other liver disorders, bone cavity filling, regeneration or repair of bone fractures, diabetes mellitus, ureter or bladder reconstruction, prolapse of the bladder or the uterus, IVF treatment, muscle wasting disorders, atrophy of the kidney, organ reconstruction and cosmetic surgery.

Described is a method of treatment comprising the administration of a scaffold or scaffold material according the description.

Described is a method of treating a subject, such as a mammalian organism, to obtain a desired local physiological or pharmacological effect comprising administering a scaffold material according to the description to a site in the subject (e.g. the organism) in need of such treatment. Preferably the method allows the agent to be delivered from the scaffold to the area surrounding the site of scaffold formation.

Described is the use of a scaffold material according to the description as an injectable scaffold material in tissue regeneration and/or in the treatment of tissue damage.

The product of the description may be used for the treatment or prevention of a condition selected from: neurodegeneration disorders (e.g. post stroke, Huntington's, Alzheimer's disease, Parkinson's disease), bone-related disorders (including osteoarthritis, spinal disk atrophy, bone cavities requiring filling, bone fractures requiring regeneration or repair), burns, cancers, liver disorders (including hepatic atrophy), kidney disorders (including atrophy of the kidney), disorders of the bladder, ureter or urethra (including damaged ureter or damaged bladder requiring reconstruction, prolapse of the bladder or the uterus), diabetes mellitus, infertility requiring IVF treatment, muscle wasting disorders (including muscular dystrophy), cardiac disorders (e.g. damaged cardiac tissue post myocardial infarction, congestive heart disease), eye disorders (e.g. damaged or diseased cornea), damaged vasculature requiring regeneration or repair, ulcers, and damaged tissue requiring regeneration or reconstruction (including damaged organ requiring regeneration or reconstruction, and damaged nerves requiring regeneration or reconstruction).

Described is a kit for use to form a scaffold comprising:
polymer microparticles; and
a carrier solution comprising the first plasticiser; and the polymer microparticles and/or the carrier comprise the second plasticiser; and further optionally instructions to mix the polymer microparticles, powdered agent and carrier.

The polymer microparticles and powdered agent may be pre-mixed. In another embodiment, the natural-polymer particles and/or non-polymer particles, polymer microparticles and powdered agent may be pre-mixed. In another embodiment, the natural-polymer particles and/or non-polymer particles, polymer microparticles, carrier and powdered agent may be pre-mixed.

The plasticiser may be provided separately for mixing with the carrier.

The kit may include a syringe for use in injecting the scaffold material. The kit may further include cells and/or active agents for mixing with the scaffold material. The kit may be stored either refrigerated or at room temperature.

References herein to the percentage being w/v may alternatively be v/v where appropriate.

The skilled man will appreciate that the optional features of the first aspect, or any aspect or embodiment, of the description can be applied to all aspects of the description.

Embodiments of the description will now be described, by way of example only, with reference to the following examples.

### EXAMPLES (not according to the claimed invention)

### 1. Description

This document discusses research into the development of smart pastes, able to set at different times and at different temperatures, to be used alone or in combination with drugs, growth factors, genes or cells. In this example, these pastes were made by two main components, PLGA or PLGA/ceramic particles and a liquid carrier.

Calcium sulphate (CS) and β-Tricalcium Phosphate (β-TCP) are the ceramic investigated. They have previously been shown to induce bone formation in vivo and act to reduce the overall cost of goods of a potential end product. It was therefore investigated if ceramics could be included for this indication and how they would affect the properties of the final product.

The present description describes a method to control the setting of said pastes by using liquid carriers with two different plasticizers (TEC and EtOH) and different concentrations of them. They have been tested with particles of different composition and size.

In this document it is claimed that when a plasticiser, such us TEC, is used in the liquid carrier, the use of PEG can be avoided. In this way, PLGA particles and not only PLGA/PEG particles could be used.

This document also claims that when a plasticiser, such us TEC, is used in the liquid carrier it is possible to sinter particles made by PLGA/ceramic blends.

Furthermore, it is claimed that by choosing the concentration of TEC it is possible to control the setting properties of the TAOS material.

### Example 1- Paste setting control over time and temperature

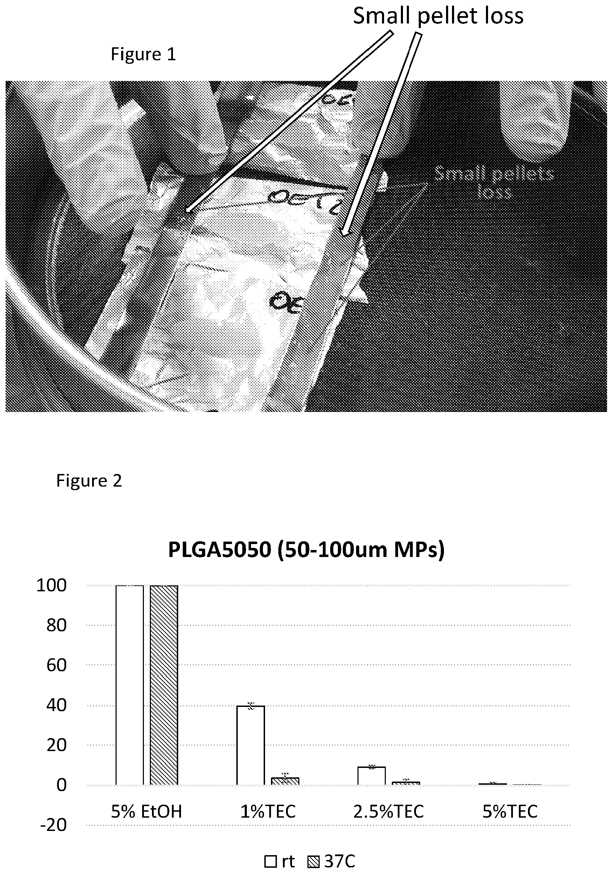

Pastes were prepared by mixing particles with a liquid carrier and their setting was assessed using an `in house' cohesion test. Briefly, following paste sintering, aluminium foils containing the pastes were placed onto a sieve mesh and immersed to a depth of around 1cm of water for 1 minute (fig.1). Afterwards, they were carefully removed from the sieve. The samples were freeze-dried and weighed so that mass loss could be estimated.

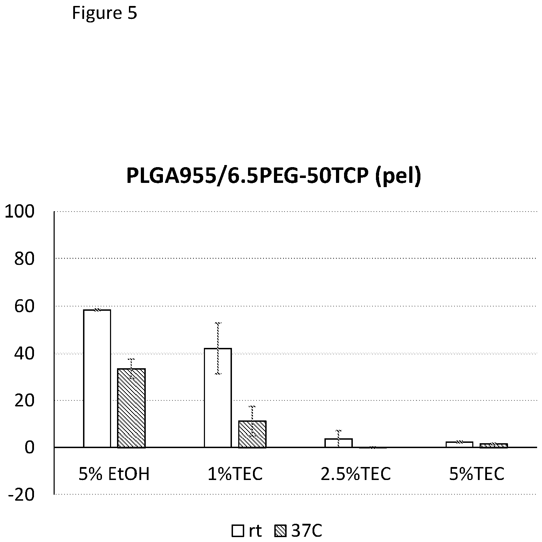

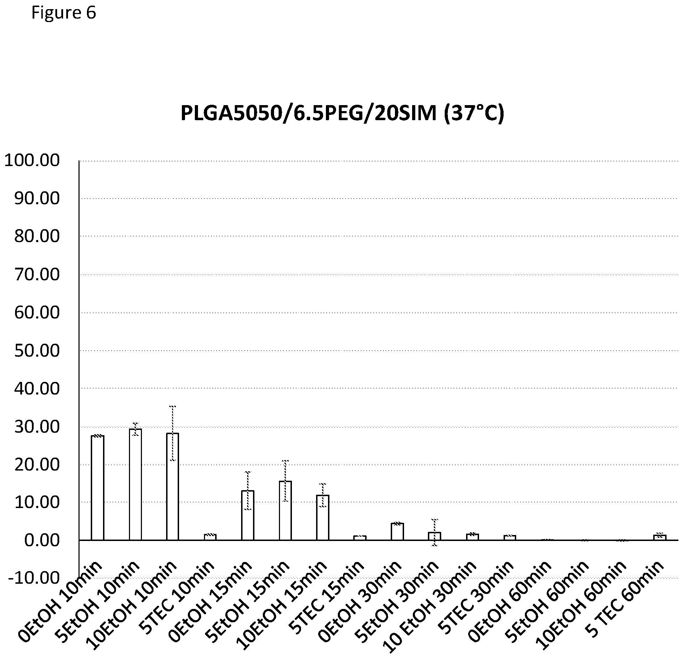

Fig. 2-5 show the mass loss from different pastes sintered for 15 min at room temperature or 37°C. Fig 6 shows the mass loss of pastes sintered at different time points (from 10 to 60 min.) at 37°C.

### Materials

### Liquid carriers:

- 0.5% w/v CMC, 1% w/v Pluronic F127 in 0.9% w/v Sodium Chloride.
- 1% w/v TEC, 0.5% w/v CMC, 1% w/v Pluronic F127 in 0.9% w/v Sodium Chloride.
- 2.5% w/v TEC, 0.5% w/v CMC, 1% w/v Pluronic F127 in 0.9% w/v Sodium Chloride.
- 5% w/v TEC, 0.5% w/v CMC, 1% w/v Pluronic F127 in 0.9% w/v Sodium Chloride.
- 5% w/v EtOH, 0.5% w/v CMC, 1% w/v Pluronic F127 in 0.9% w/v Sodium Chloride.
- 10% w/v EtOH, 0.5% w/v CMC, 1% w/v Pluronic F127 in 0.9% w/v Sodium Chloride.

### Particles:

- PLGA 50:50 (50-100µm MPs).
- 75.6% w/w PLGA50:50, 5.2% w/w PEG400, 20% w/w SIM (300-400µm HME pellets).
- 46.5% w/w PLGA 95:5, 3.25% w/w PEG400, 50% w/w CS (300-400µm HME pellets).
- 46.5% w/w PLGA 95:5, 3.25% w/w PEG400, 50% w/w β-TCP (300-400µm HME pellets).

### Method

- A 355µm sieve (Endecotts, BS410/1986) was placed on to its dedicated collection tray.
   - 2 x 100mg of particles were manually mixed in an aluminium foil (4x4cm circa) with 70µl of each liquid carrier.
   - The obtained pastes were left to sinter in a sealed plastic bag with humidity > 90% for different times.
- Following sintering at RT or 37°C in a humidified environment (>90% RH), the aluminium foils containing the pastes were placed onto the sieve mesh.
- A constant, gentle, circa 7ml/sec flow of water (Millipore, Direct-Q^{®} 3 UV) was applied to the sieve mesh, until the samples became immersed in circa 1cm of water.
- Following immersion, samples were allowed to remain immersed in the head of water for circa 1 minute.
- After the 1 minute, the sieve was removed from the sieve tray and the aluminium foils containing the samples were carefully removed from the sieve.
- The samples with the aluminium foil were freeze-dried and weighed so that mass loss could be estimated.
- The sieve tray (which was still filled with water) was visually inspected for the presence of particles that may have been lost from the samples during.

Fig. 2 shows that using a liquid carrier containing a plasticizer, it is possible to use PLGA particles that are not blended with the plasticizer PEG400. This is important because removing the plasticizer from the blend will give a leaner manufacturing process and improve the room temperature stability of the particles. In fact, because of the low glass transition temperatures of PLGA/PEG400 blends, they need to be stored in fridge or freezer.

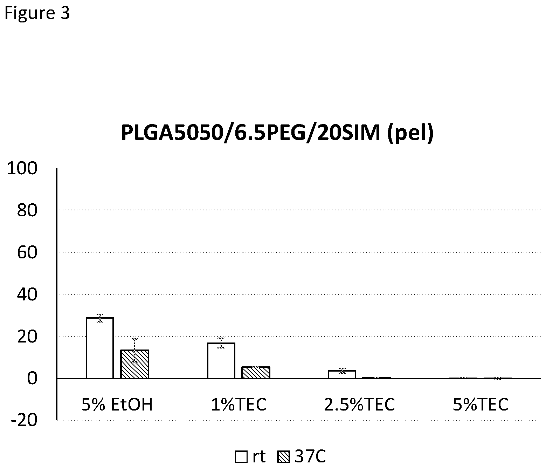

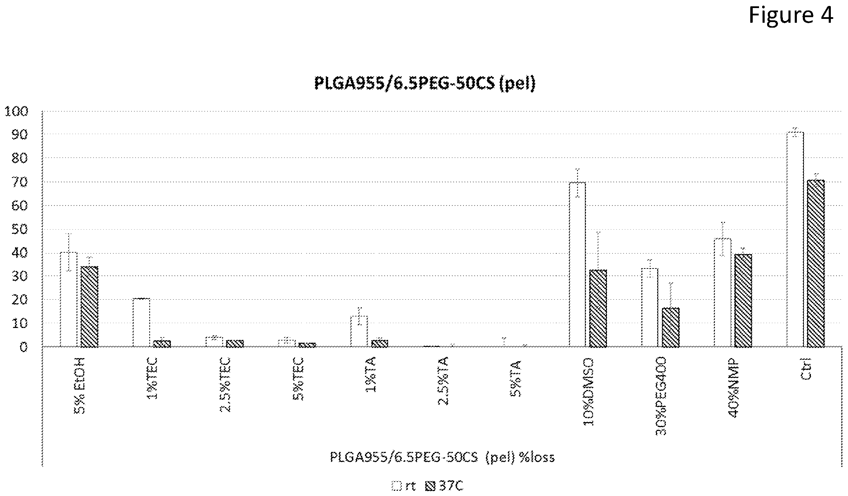

Fig 2-5 demonstrates that increasing the TEC concentration in the liquid carrier produces pastes with fast setting properties. Except for the 20%SIM particles (fig.3), the liquid carrier containing 1% TEC gave pastes able to set at 37°C and not at room temperature. Instead, with the liquid carrier containing 5% TEC, pastes were obtained which were very fast setting and had a putty-like consistency. The same setting conditions with different liquid carriers containing the plasticisers DMSO, PEG400, NMP or triacetin (TA) is also shown in Fig. 4. Fig.4 demonstrates that TEC and TA behave in a similar way and that cohesion can be controlled not only by changing the plasticiser concentration but also by choosing a different plasticiser.

Fig. 6 shows how the paste cohesion is affected by time and that the presence of ethanol in the liquid carrier is not important for paste setting.

### Example 2 - Paste mechanical property control over time and temperature (strength)

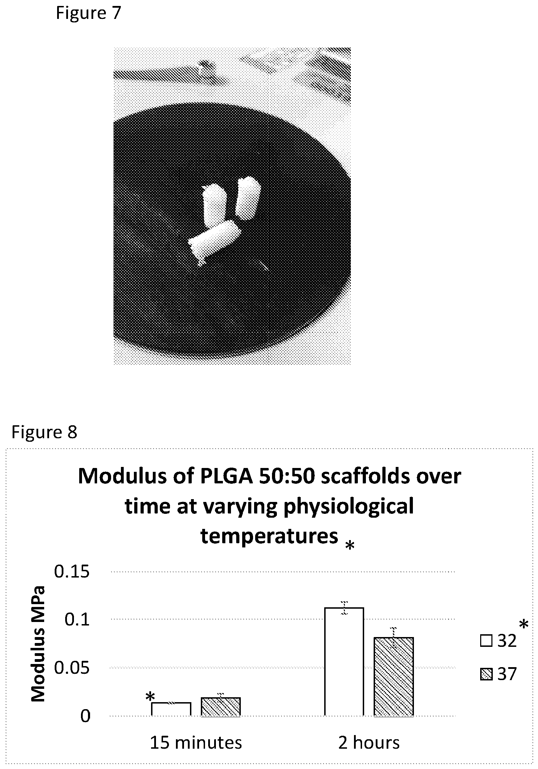

Mechanical properties of the paste-formed scaffolds were assessed. Scaffold strength of 6x12 mm cylindrical scaffolds (fig.7) was assessed after 15min, 2 and 24h sintering, using a `Stable Microsystems' texture analyser following the Locate Therapeutics testing protocol. PLGA or PLGA/CS particles were investigated.

### Materials

### Liquid carriers:

- 3% w/v TEC, 0.5% w/v CMC, 1% w/v Pluronic F127 in 0.9% w/v Sodium Chloride.

### Particles:

- PLGA 50:50 (50-200µm MPs).
- 50% w/w PLGA50:50, 50% w/w CS (50-200µm MPs).

### Method

6x12 mm cylindrical scaffolds were produced by syringe mixing PLGA or PLGA/CS (50-200 µm) MPs with 3% TEC carrier at a 1.5:1 ratio, injecting to a PTFE mould and sintering at either 32°C or 37°C for 15 minutes and 2 hours or 24 hours either immersed in PBS (wet) or sealed in a >90% humid atmosphere at 37°C (fig.7). Mechanical testing was carried out according to ISO standard by using a texture analyser.

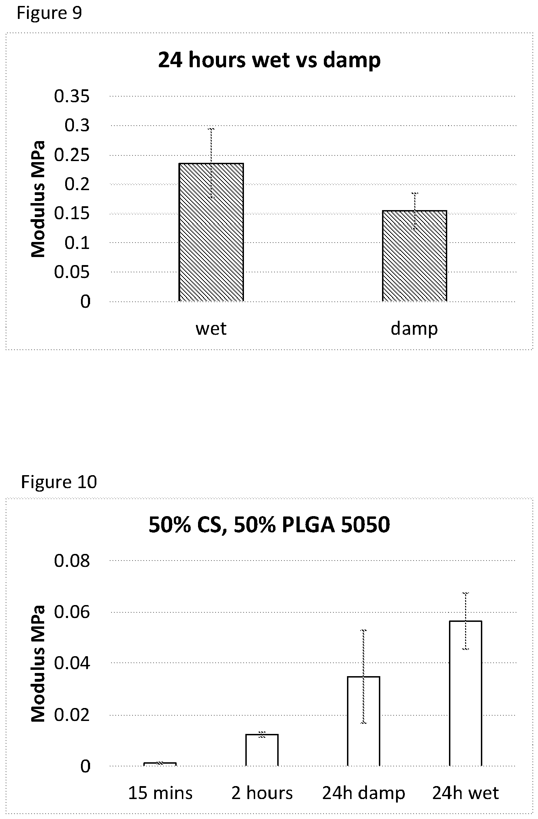

The obtained results demonstrate that scaffolds were formed after 15 min sintering using both PLGA and PLGA/CS MPs. Nevertheless, the addition of CS resulted in weaker scaffolds (fig.8 and fig. 10).

When PLGA5050 was used, 15 min sintering at 32°C or 37°C has no effect on scaffold strength. Instead, 2h sintering gave strongest scaffolds at 32°C (Fig. 8).

Comparison between damp and wet sintering (fig. 9-10) demonstrates that the strongest scaffolds are those sintered in wet conditions.

### Example 3 - Paste mechanical property control over time and temperature (flow ability)

### Materials

### Liquid carriers:

- 2% w/v TEC, 0.5% w/v CMC, 1% w/v Pluronic F127 in 0.9% w/v Sodium Chloride.
- 3% w/v TEC, 0.5% w/v CMC, 1% w/v Pluronic F127 in 0.9% w/v Sodium Chloride.
- 4% w/v TEC, 0.5% w/v CMC, 1% w/v Pluronic F127 in 0.9% w/v Sodium Chloride.
- 10% w/v EtOH, 0.5% w/v CMC, 1% w/v Pluronic F127 in 0.9% w/v Sodium Chloride.

### Particles:

- PLGA 50:50 (50-200µm MPs).
- 50% w/w PLGA50:50, 50% w/w CS (50-200µm MPs).

### Method

Viscosity was determined by ejecting 400µL mixed putty onto an acetate sheet set to an angle of 45°. Putty was ejected directly from the syringe at a steady rate, and allowed to flow down the slope for 60 seconds before a second mark was made to indicate how far the putty had run. The distance was then calculated and average values obtained.

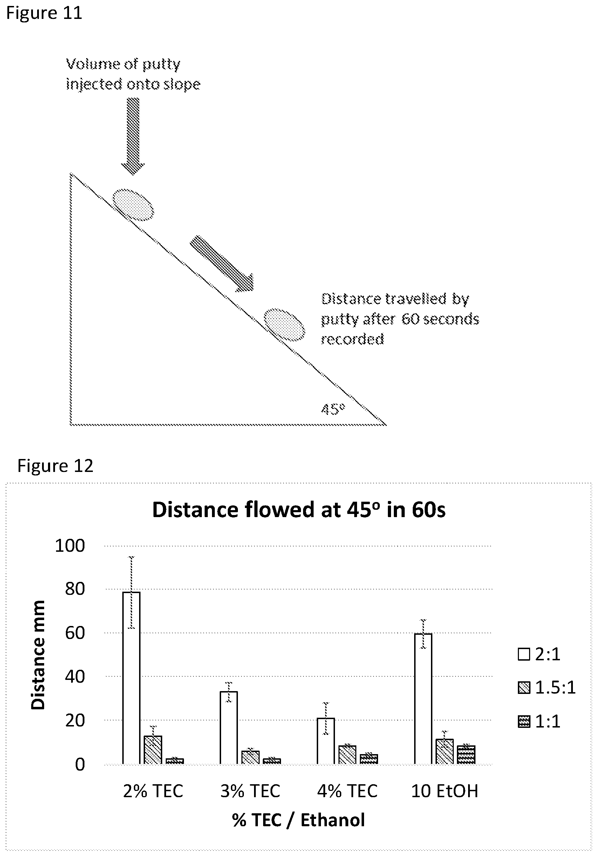

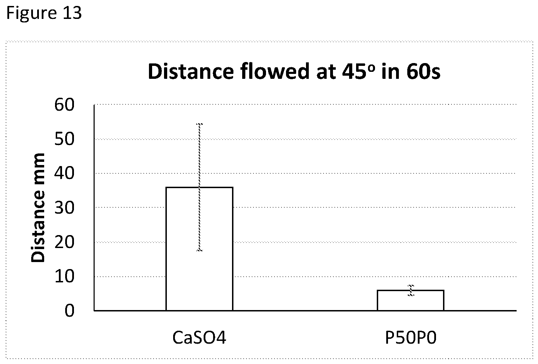

The results show that by reducing the amount of carrier to polymer ratio before mixing, a much thicker paste can be generated, regardless of the carrier components. By increasing plasticizer concentration it is possible to create a more viscous initial material, but only to a limit dependent on the material, after which further increases in plasticiser make little difference (fig. 12). Further results demonstrate that the addition of calcium sulphate has a great impact on the flowability of mixed and ejected material (fig. 13).

This technology can be exploited in the medical healthcare area. Applications include an injectable delivery system for use in cellular therapies which encourages the formation of 3D tissue structures to give enhanced functionality e.g. dental, bone defects, bone fractures, spine fusion and cartilage. The market for orthopaedic materials is vast, and growing in line with the aging population. As such, novel, cost effective therapy products are vital in maintaining healthcare standards and keeping costs to reasonable levels.

### 2. Terminology

β-TCP: β-tricalcium phosphate, CMC: Carboxymethyl cellulose, CS: Calcium suplphate, EtOH: Ethanol, F127: Pluronic^{®} F127, HME: Hot Melt Extrusion, MPs: Microparticles, PEG: Polyethene glycol, PLGA: Poly(lactic-co-glycolic acid), TEC: TriEthyl Citrate

### References

1- M. Artico, L. Ferrante, F. S. Pastore et al., "Bone autografting of the calvaria and craniofacial skeleton: historical background, surgical results in a series of 15 patients, and review of the literature," Surgical Neurology, vol. 60, no. 1, pp. 71-79, 2003.
2- Y. T. Konttinen, D. Zhao, A. Beklen et al., "The microenvironment around total hip replacement prostheses," Clinical Orthopaedics and Related Research, no. 430, pp. 28-38, 2005.
3- L. G. Mercuri and A. Giobbie-Hurder, "Long-term outcomes after total alloplastic temporomandibular joint reconstruction following exposure to failed materials," Journal of Oral and Maxillofacial Surgery, vol. 62, no. 9, pp. 1088-1096, 2004.
4- A. M. Pou, "Update on new biomaterials and their use in reconstructive surgery," Current Opinion in Otolaryngology and Head and Neck Surgery, vol. 11, no. 4, pp. 240-244, 2003.
5- R. Langer and J. P. Vacanti, "Tissue engineering," Science, vol. 260, no. 5110, pp. 920-926, 1993.

### Example Scaffold Properties

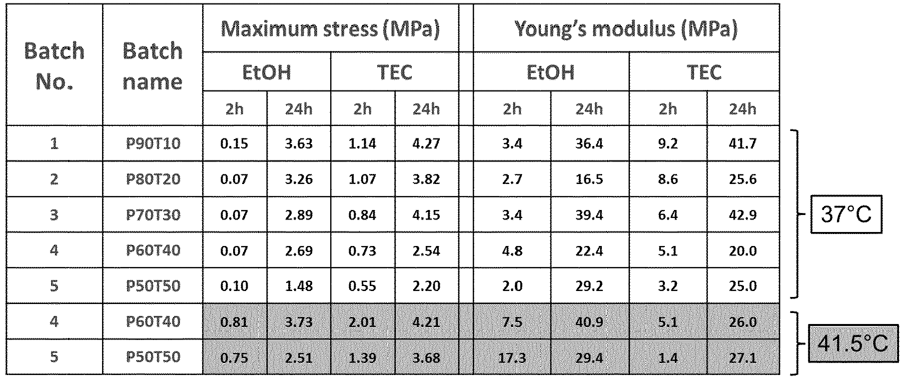

In `batch name' column, P and T refer to PLGA/PEG and β-TCP respectively.

EtOH and TEC were used as plasticisers in the liquid carrier at the concentration of 5% and 2.5% respectively.

### Example - TAOS^{™} microparticles production by HME process

### Summary

This example describes a general protocol to produce TAOS^{™} microparticles by using the hot melt extrusion technique.

TAOS^{™} is a PLGA-based material that can be blended with biodegradable and biocompatible material such as ceramics (i.e. Calcium sulphate, tricalcium phosphate) and polymers (i.e. PEGs, Pluronics, etc). TAOS^{™} can deliver active ingredients and biologicals.

Each blend is composed at least by two components where one component is PLGA. The methodology described in this document consist of 3 main steps:
- Dry pre-mixing of the materials
- Hot melt extrusion of the pre-mixed material
- Pelletisation of the extrudate

Using this protocol microparticles of 300-400microns are obtained. When smallest particles are required, the further step of cryomilling is required.

### - Materials

PLGA 50:50 4.5A, Evonik, Lot No. LP1042
PLGA 85:15 4A, Evonik, Lot No. LP717
PLGA 95:5 6E, Evonik, Lot No. LP1075
Pluronic F127, Sigma, Lot no 121K0070
PEG 400, Clariant, Lot No. DEG4242071
Simvastatin, Teva, Lot No. 86600300414
Calcium Sulphate (CS), Sigma-Aldrich, Lot No. MKBR2597V
β-TCP, Plasma-Biotal, Lot No. XRD7065

### - Method

### Dry pre-mixing

Mixing is performed by hand in triple bagged samples of at least 30g, breaking up larger particles as they are identified and remixing into the blend. Once finding agglomerates to break down became hard, the blend is passed through an 850microns screen with particles separated out in this process broken down and recombined with the main material feedstock. The recombined feedstock is then thoroughly mixed ready for hot melt extrusion. In this manner, consistent feeding of each material is achieved using a twin screw mini-feeder into the hot melt extruder.

Agglomerates occur when PEG400 or other sticky liquid materials are used in combination with PLGA.

PLGA, simvastatin and Pluronic F127 should be less than 500 microns prior to use.

The BTCP and the CS should be between 20 and 52 microns.

### - Hot melt extrusion

The pre-mixed material is then introduced into the HME feeding zone through a twin screw mini-feeder. Varying the feed rates of the material to the extruder, from 1.1 to 2.3 g min⁻¹, it is possible to change the extrudate diameter from 300-350microns to 800-900microns.

Standard HME barrel temperatures are shown below. The extruder screw speed is set at 50 rpm.

Barrel temperature profiles:

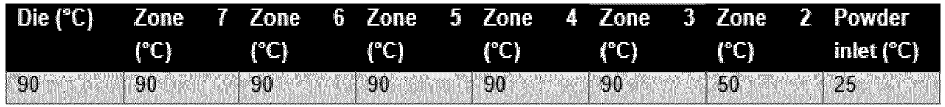

### - Pelletisation

The extrudate coming from the HME die zone is then cut into microparticles using a pelletiser with the setting 9L1. With this setting particles of 300-400microns are obtained.

The pelletiser is composed by 2 main components:
- 1 cutting wheel
- 2 concentric wheels that push the extrudate to the cutting wheel.

The cutting wheel can rotate at different speed (control dial from 1 to 9). Instead, the 2 concentric wheels can push the extrudate at four different speeds (control dial from 1 to 4). In the combination 9L1, 9 refers to the cutting wheel while 1 refers to the concentric wheels.

### Prepared batches

Using the described methodology following HME batches were produced:
-TAOS-TCP
- PLGA 95:5, PEG 400 (6.5%), TCP (10%)
- PLGA 95:5, PEG 400 (6.5%), TCP (20%)
- PLGA 95:5, PEG 400 (6.5%), TCP (30%)
- PLGA 95:5, PEG 400 (6.5%), TCP (40%)
- PLGA 95:5, PEG 400 (6.5%), TCP (50%)

-TAOS-TCP/TAOS-CS/TAOS-Pluronic
- Batch LOC01 - PLGA 95:5, PEG 400 (6.5%), TCP (50%)
- Batch LOC02 - PLGA 95:5, PEG 400 (6.5%), CS (50%)
- Batch LOC03 - PLGA 50:50, PEG 400 (6.5%), Pluronic F127 (10%)
- Batch LOC04 - PLGA 95:5, PEG 400 (6.5%), Pluronic F127 (10%), Simvastatin (20%)

-TAOS-SIM

| **Batch Output** | **Polymer** | **PEG 400 (w.r.t. PLGA)** | **Simvastatin** | **Feed Rate** | **Pelletiser Rate** |
|---|---|---|---|---|---|
| 200g | PLGA 9.5:5 | 6.5% | 0 % | 1.1g/min | 9L1 |
| 200g | PLGA 50:50 | 6.5% | 20% | 1.1g/min | 9L1 |
| 200g | PLGA 50:50 | 6.5% | 0 % | 1.1g/min | 9L1 |
| 200g | PLGA 85:15 | 6.5% | 0 % | 1.1g/min | 9L1 |
| 100g | PLGA 95:5 | 6.5% | 20% | 1.1g/min | 9L1 |
| 100g | PLGA 85:15 | 6.5% | 20% | 1.1g/min | 9L1 |

## Claims

1. A method of forming a scaffold material *in vitro,* the method comprising:
providing polymer microparticles;
suspending the polymer microparticles in a liquid carrier to form a scaffold material, which is a polymer microparticle suspension, wherein the scaffold material comprises a first plasticiser and a second plasticiser in the liquid carrier,
wherein the first plasticiser is selected from any one of TEC (triethyl citrate), alcohol, benzoic acid, triacetin, NMP, DMSO and PEG; and
the second plasticiser is TEC (triethyl citrate) or triacetin (TA), wherein the first and second plasticisers are different.

2. The method according to claim 1, further comprising the step of setting the polymer microparticle suspension such that it sets into a solid scaffold of polymer microparticles.

3. A scaffold material for forming a scaffold, wherein the scaffold material comprises:
polymer microparticles;
a liquid carrier suspending the polymer microparticles,
wherein the scaffold material comprises a first plasticiser and a second plasticiser in the liquid carrier,
wherein if the first plasticiser is selected from any one of TEC (triethyl citrate), alcohol, benzoic acid, triacetin, NMP, DMSO and PEG; and
the second plasticiser is TEC (triethyl citrate) or triacetin (TA), wherein the first and second plasticisers are different.

4. The scaffold material according to claim 3 for use in a method of treatment requiring delivery of an agent to a subject, wherein the agent is located within polymer microparticles within the scaffold material; wherein the method of treatment comprises administering the scaffold material to a subject; allowing the scaffold material to solidify/self-assemble in the subject to form a scaffold; and allowing the agent contained within the scaffold material to be released into the subject at the site of administration.

5. A kit for use to form a scaffold from scaffold material comprising:
polymer microparticles; and
a carrier solution comprising a first and second plasticiser,
wherein the first plasticiser is selected from any one of TEC (triethyl citrate), alcohol, benzoic acid, triacetin, NMP, DMSO and PEG; and
the second plasticiser is TEC (triethyl citrate) or triacetin (TA), wherein the first and second plasticisers are different.

6. The method according to claims 1 or 2, the scaffold material according to claim 3, the scaffold material for the use according to claim 4, or the kit according to claim 5, wherein the scaffold of polymer microparticles is porous,
optionally wherein the scaffold has a pore volume of at least about 50%.

7. The method according to any one of claims 1, 2 or 6, the scaffold material according to any one of claims 3 or 6, the scaffold material for the use according to any one of claims 4 or 6, or the kit according to any one of claims 5 or 6, wherein the scaffold material is injectable prior to setting.

8. The method according to any one of claims 1, 2, 6 or 7, the scaffold material according to any one of claims 3, 6 or 7, the scaffold material for the use according to any one of claims 4, 6 or 7, or the kit according to any one of claims 5, 6 or 7, wherein when the polymer microparticles come together and cross-link/inter-link, pores are formed in the resultant scaffold, as a consequence of the inevitable spaces between adjacent polymer microparticles.

9. The scaffold material for the use according to any one of claims 4 or 6-8, wherein the scaffold is formed *ex situ;* or
wherein the setting of the scaffold material to form the scaffold is *in situ.*

10. The method according to any one of claims 1, 2 or 6-9, or the scaffold material for the use according to any one of claims 4 or 6-9, wherein the scaffold material is spread into a film prior to setting.

11. The method according to any one of claims 1, 2 or 6-10, the scaffold material according to any one of claims 3 or 6-8, the scaffold material for the use according to any one of claims 4 or 6-10, or the kit according to any one of claims 5 or 6-8, wherein the scaffold material comprises natural-polymer particles and/or non-polymer particles,
optionally wherein the non-polymer particles comprise or consist of ceramic, and/or wherein the natural-polymer or non-polymer particles are encapsulated within the polymer-microparticles.

12. The method according to any one of claims 1, 2 or 6-11, the scaffold material according to any one of claims 3, 6-8 or 11, the scaffold material for the use according to any one of claims 4 or 6-11, or the kit according to any one of claims 5, 6-8 or 11, wherein the polymer microparticles are substantially free of PEG,
optionally wherein the polymer microparticles have a size in their longest dimension of between 300 and 500 µm; or between 20 µm and 100 µm.

13. The method according to any one of claims 1, 2 or 6-12, the scaffold material according to any one of claims 3, 6-8, 11 or 12, the scaffold material for the use according to any one of claims 4 or 6-12, or the kit according to any one of claims 5, 6-8, 11 or 12, wherein the plasticiser in the carrier does not comprise PEG.

14. The method according to any one of claims 1, 2 or 6-13, the scaffold material according to any one of claims 3, 6-8 or 11-13, the scaffold material for the use according to claims 4 or 6-13, or the kit according to any one of claims 5, 6-8 or 11-13, wherein the ratio of carrier to polymer microparticles in the scaffold material is at least 1.5:1 (v/w).

15. The method according to any one of claims 1, 2 or 6-14, the scaffold material according to any one of claims 3, 6-8 or 11-14, the scaffold material for the use according to claims 4 or 6-14, or the kit according to any one of claims 5, 6-8 or 11-14, wherein viable cells are provided in the scaffold material.

## Patentansprüche

1. Verfahren zum Bilden eines Gerüstmaterials in vitro, wobei das Verfahren Folgendes umfasst:
Bereitstellen von Polymermikropartikeln;
Suspendieren der Polymermikropartikel in einem flüssigen Träger, um ein Gerüstmaterial zu bilden, das eine Polymermikropartikelsuspension ist, wobei das Gerüstmaterial einen ersten Weichmacher und einen zweiten Weichmacher in dem flüssigen Träger umfasst,
wobei der erste Weichmacher aus einem beliebigen von TEC (Triethylcitrat), Alkohol, Benzoesäure, Triacetin, NMP, DMSO und PEG ausgewählt ist; und
der zweite Weichmacher TEC (Triethylcitrat) oder Triacetin (TA) ist, wobei sich der erste und der zweite Weichmacher unterscheiden.

2. Verfahren nach Anspruch 1, ferner umfassend den Schritt des Erhärtens der Polymermikropartikelsuspension, sodass sie sich zu einem festen Gerüst aus Polymermikropartikeln erhärtet.

3. Gerüstmaterial zum Bilden eines Gerüsts, wobei das Gerüstmaterial Folgendes umfasst:
Polymermikropartikel;
einen flüssigen Träger, der die Polymermikropartikel suspendiert,
wobei das Gerüstmaterial einen ersten Weichmacher und einen zweiten Weichmacher in dem flüssigen Träger umfasst,
wobei der erste Weichmacher aus einem beliebigen von TEC (Triethylcitrat), Alkohol, Benzoesäure, Triacetin, NMP, DMSO und PEG ausgewählt ist; und
der zweite Weichmacher TEC (Triethylcitrat) oder Triacetin (TA) ist, wobei sich der erste und der zweite Weichmacher unterscheiden.

4. Gerüstmaterial nach Anspruch 3 zur Verwendung in einem Behandlungsverfahren, das Abgabe eines Mittels an ein Subjekt erfordert, wobei sich das Mittel in Polymermikropartikeln innerhalb des Gerüstmaterials befindet; wobei das Behandlungsverfahren Verabreichen des Gerüstmaterials an ein Subjekt; Ermöglichen, dass sich das Gerüstmaterial in dem Subjekt verfestigt/selbst zusammensetzt, um ein Gerüst zu bilden; und Ermöglichen, dass das in dem Gerüstmaterial enthaltene Mittel an der Verabreichungsstelle an das Subjekt freigesetzt wird, umfasst.

5. Kit zur Verwendung zum Bilden eines Gerüsts aus Gerüstmaterial, umfassend:
Polymermikropartikel; und
eine Trägerlösung, umfassend einen ersten und einen zweiten Weichmacher,
wobei der erste Weichmacher aus einem beliebigen von TEC (Triethylcitrat), Alkohol, Benzoesäure, Triacetin, NMP, DMSO und PEG ausgewählt ist; und
der zweite Weichmacher TEC (Triethylcitrat) oder Triacetin (TA) ist, wobei sich der erste und der zweite Weichmacher unterscheiden.

6. Verfahren nach Anspruch 1 oder 2, Gerüstmaterial nach Anspruch 3, Gerüstmaterial zur Verwendung nach Anspruch 4 oder Kit nach Anspruch 5, wobei das Gerüst aus Polymermikropartikeln porös ist,
wobei das Gerüst optional ein Porenvolumen von mindestens etwa 50 % aufweist.

7. Verfahren nach einem der Ansprüche 1, 2 oder 6, Gerüstmaterial nach einem der Ansprüche 3 oder 6, Gerüstmaterial zur Verwendung nach einem der Ansprüche 4 oder 6 oder Kit nach einem der Ansprüche 5 oder 6, wobei das Gerüstmaterial vor dem Erhärten injizierbar ist.

8. Verfahren nach einem der Ansprüche 1, 2, 6 oder 7, Gerüstmaterial nach einem der Ansprüche 3, 6 oder 7, Gerüstmaterial zur Verwendung nach einem der Ansprüche 4, 6 oder 7 oder Kit nach einem der Ansprüche 5, 6 oder 7, wobei, wenn die Polymermikropartikel zusammenkommen und sich untereinander vernetzen/miteinander vernetzen, in dem resultierenden Gerüst als Folge der unvermeidbaren Räume zwischen benachbarten Polymermikropartikeln Poren gebildet werden.

9. Gerüstmaterial zur Verwendung nach einem der Ansprüche 4 oder 6-8, wobei das Gerüst ex situ gebildet ist; oder
wobei das Erhärten des Gerüstmaterials zum Bilden des Gerüsts in situ erfolgt.

10. Verfahren nach einem der Ansprüche 1, 2 oder 6-9 oder Gerüstmaterial zur Verwendung nach einem der Ansprüche 4 oder 6-9, wobei das Gerüstmaterial vor dem Erhärten in einen Film verteilt wird.

11. Verfahren nach einem der Ansprüche 1, 2 oder 6-10, Gerüstmaterial nach einem der Ansprüche 3 oder 6-8, Gerüstmaterial zur Verwendung nach einem der Ansprüche 4 oder 6-10 oder Kit nach einem der Ansprüche 5 oder 6-8, wobei das Gerüstmaterial natürliche Polymerpartikel und/oder Nichtpolymerpartikel umfasst,
wobei die Nichtpolymerpartikel optional Keramik umfassen oder daraus bestehen und/oder wobei die natürlichen Polymerpartikel oder Nichtpolymerpartikel in den Polymermikropartikeln eingekapselt sind.

12. Verfahren nach einem der Ansprüche 1, 2 oder 6-11, Gerüstmaterial nach einem der Ansprüche 3, 6-8 oder 11, Gerüstmaterial für die Verwendung nach einem der Ansprüche 4 oder 6-11 oder Kit nach einem der Ansprüche 5, 6-8 oder 11, wobei die Polymermikropartikel im Wesentlichen frei von PEG sind,
wobei die Polymermikropartikel optional eine Größe in ihrer längsten Abmessung zwischen 300 und 500 µm; oder zwischen 20 µm und 100 µm aufweisen.

13. Verfahren nach einem der Ansprüche 1, 2 oder 6-12, Gerüstmaterial nach einem der Ansprüche 3, 6-8, 11 oder 12, Gerüstmaterial zur Verwendung nach einem der Ansprüche 4 oder 6-12 oder Kit nach einem der Ansprüche 5, 6-8, 11 oder 12, wobei der Weichmacher in dem Träger kein PEG umfasst.

14. Verfahren nach einem der Ansprüche 1, 2 oder 6-13, Gerüstmaterial nach einem der Ansprüche 3, 6-8 oder 11-13, Gerüstmaterial für die Verwendung nach den Ansprüchen 4 oder 6-13 oder Kit nach einem der Ansprüche 5, 6-8 oder 11-13, wobei das Verhältnis von Träger zu Polymermikropartikeln in dem Gerüstmaterial mindestens 1,5:1 (v/w) beträgt.

15. Verfahren nach einem der Ansprüche 1, 2 oder 6-14, Gerüstmaterial nach einem der Ansprüche 3, 6-8 oder 11-14, Gerüstmaterial zur Verwendung nach Anspruch 4 oder 6-14 oder Kit nach einem der Ansprüche 5, 6-8 oder 11-14, wobei lebensfähige Zellen in dem Gerüstmaterial bereitgestellt sind.

## Revendications

1. Procédé de formation d'un matériau d'échafaudage in vitro, le procédé comprenant :
la fourniture de microparticules de polymère ;
la mise en suspension des microparticules de polymère dans un véhicule liquide pour former un matériau d'échafaudage, qui est une suspension de microparticules de polymère, dans lequel le matériau d'échafaudage comprend un premier plastifiant et un second plastifiant dans le véhicule liquide,
dans lequel le premier plastifiant est choisi parmi l'un quelconque du TEC (citrate de triéthyle), d'un alcool, de l'acide benzoïque, de la triacétine, de la NMP, du DMSO and du PEG ; et
le second plastifiant est du TEC (citrate de triéthyle) ou de la triacétine (TA), dans lequel les premier et second plastifiants sont différents.

2. Procédé selon la revendication 1, comprenant en outre l'étape de solidification de la suspension de microparticules de polymère de telle sorte qu'elle se solidifie en un échafaudage solide de microparticules de polymère.

3. Matériau d'échafaudage permettant de former un échafaudage, dans lequel le matériau d'échafaudage comprend :
des microparticules de polymère ;
un véhicule liquide permettant la mise en suspension des microparticules de polymère,
dans lequel le matériau d'échafaudage comprend un premier plastifiant et un second plastifiant dans le véhicule liquide,
dans lequel le premier plastifiant est choisi parmi l'un quelconque du TEC (citrate de triéthyle), d'un alcool, de l'acide benzoïque, de la triacétine, de la NMP, du DMSO and du PEG ; et
le second plastifiant est du TEC (citrate de triéthyle) ou de la triacétine (TA), dans lequel les premier et second plastifiants sont différents.

4. Matériau d'échafaudage selon la revendication 3 destiné à être utilisé dans un procédé de traitement nécessitant l'administration d'un agent à un sujet, dans lequel l'agent est situé à l'intérieur de microparticules de polymère à l'intérieur du matériau d'échafaudage ; dans lequel le procédé de traitement comprend l'administration du matériau d'échafaudage à un sujet ; le fait de laisser le matériau d'échafaudage se solidifier/s'auto-assembler dans le corps du sujet pour former un échafaudage ; et le fait de laisser l'agent contenu dans le matériau d'échafaudage se libérer dans le corps du sujet au site d'administration.

5. Kit destiné à être utilisé pour la formation d'un échafaudage à partir d'un matériau d'échafaudage comprenant :
des microparticules de polymère ; et
une solution véhicule comprenant des premier et second plastifiants,
dans lequel le premier plastifiant est choisi parmi l'un quelconque du TEC (citrate de triéthyle), d'un alcool, de l'acide benzoïque, de la triacétine, de la NMP, du DMSO and du PEG ; et
le second plastifiant est du TEC (citrate de triéthyle) ou de la triacétine (TA), dans lequel les premier et second plastifiants sont différents.

6. Procédé selon les revendications 1 ou 2, matériau d'échafaudage selon la revendication 3, matériau d'échafaudage destiné à être utilisé selon la revendication 4 ou kit selon la revendication 5, où l'échafaudage de microparticules de polymère est poreux,
où éventuellement l'échafaudage a un volume de pores d'au moins environ 50 %.

7. Procédé selon l'une quelconque des revendications 1, 2 ou 6, matériau d'échafaudage selon l'une quelconque des revendications 3 ou 6, matériau d'échafaudage destiné à être utilisé selon l'une quelconque des revendications 4 ou 6 ou kit selon l'une quelconque des revendications 5 ou 6, où le matériau d'échafaudage est injectable avant la solidification.

8. Procédé selon l'une quelconque des revendications 1, 2, 6 ou 7, matériau d'échafaudage selon l'une quelconque des revendications 3, 6 ou 7, matériau d'échafaudage destiné à être utilisé selon l'une quelconque des revendications 4, 6 ou 7 ou kit selon l'une quelconque des revendications 5, 6 ou 7, où lorsque les microparticules de polymère s'assemblent et se réticulent/se lient entre elles, des pores se forment dans l'échafaudage résultant, en raison des espaces inévitables entre des microparticules de polymère adjacentes.

9. Matériau d'échafaudage destiné à être utilisé selon l'une quelconque des revendications 4 ou 6 à 8, dans lequel l'échafaudage est formé ex situ ; ou
dans lequel la solidification du matériau d'échafaudage pour former l'échafaudage se passe in situ.

10. Procédé selon l'une quelconque des revendications 1, 2 ou 6 à 9 ou matériau d'échafaudage destiné à être utilisé selon l'une quelconque des revendication 4 ou 6 à 9, où le matériau d'échafaudage est étalé en un film avant solidification.

11. Procédé selon l'une quelconque des revendications 1, 2 ou 6 à 10, matériau d'échafaudage selon l'une quelconque des revendications 3 ou 6 à 8, matériau d'échafaudage destiné à être utilisé selon l'une quelconque des revendications 4 ou 6 à 10 ou kit selon l'une quelconque des revendications 5 ou 6 à 8, où le matériau d'échafaudage comprend des particules de polymère naturel et/ou des particules non polymères,
où éventuellement les particules non polymères comprennent ou sont constituées de céramique, et/ou où les particules de polymère naturel ou non polymères sont encapsulées dans les microparticules de polymère.

12. Procédé selon l'une quelconque des revendications 1, 2 ou 6 à 11, matériau d'échafaudage selon l'une quelconque des revendications 3, 6 à 8 ou 11, matériau d'échafaudage destiné à être utilisé selon l'une quelconque des revendications 4 ou 6 à 11 ou kit selon l'une quelconque des revendications 5, 6 à 8 ou 11, où les microparticules de polymère sont sensiblement dépourvues de PEG,
où éventuellement les microparticules de polymère ont une taille dans leur dimension la plus longue comprise entre 300 et 500 µm ; ou entre 20 µm et 100 µm.

13. Procédé selon l'une quelconque des revendications 1, 2 ou 6 à 12, matériau d'échafaudage selon l'une quelconque des revendications 3, 6 à 8, 11 ou 12, matériau d'échafaudage destiné à être utilisé selon l'une quelconque des revendications 4 ou 6 à 12 ou kit selon l'une quelconque des revendications 5, 6 à 8, 11 ou 12, où le plastifiant dans le véhicule ne comprend pas de PEG.

14. Procédé selon l'une quelconque des revendications 1, 2 ou 6 à 13, matériau d'échafaudage selon l'une quelconque des revendications 3, 6 à 8 ou 11 à 13, matériau d'échafaudage destiné à être utilisé selon l'une quelconque des revendications 4 ou 6 à 13 ou kit selon l'une quelconque des revendications 5, 6 à 8 ou 11 à 13, où le rapport du véhicule aux microparticules de polymère dans le matériau d'échafaudage est d'au moins 1,5:1 (v/p).

15. Procédé selon l'une quelconque des revendications 1, 2 ou 6 à 14, matériau d'échafaudage selon l'une quelconque des revendications 3, 6 à 8 ou 11 à 14, matériau d'échafaudage destiné à être utilisé selon l'une quelconque des revendications 4 ou 6 à 14 ou kit selon l'une quelconque des revendications 5, 6 à 8 ou 11 à 14, où des cellules viables sont fournies dans le matériau d'échafaudage.
